(19) **Europäisches Patentamt**
**European Patent Office**
**Office européen des brevets**

(11) **EP 4 678 052 A1**

(12) **EUROPEAN PATENT APPLICATION**
published in accordance with Art. 153(4) EPC

(43) Date of publication:
**14.01.2026 Bulletin 2026/03**

(21) Application number: **24767018.5**

(22) Date of filing: **29.02.2024**

(51) International Patent Classification (IPC):
*A42B 3/28* (2006.01)    *A42B 3/04* (2006.01)
*F04D 17/04* (2006.01)

(52) Cooperative Patent Classification (CPC):
**A42B 3/28; A42B 3/04; A61B 5/4266;**
**A61B 5/4875; A61B 5/6803; F04D 17/04;**
A61B 2560/0242

(86) International application number:
**PCT/JP2024/007563**

(87) International publication number:
**WO 2024/185648 (12.09.2024 Gazette 2024/37)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **04.03.2023 JP 2023033262**

(71) Applicants:
• **Public University Corporation Suwa University of**
**Science Foundation**
**Chino-shi Nagano, 391-0292 (JP)**

• **Canal Water Co., Ltd.**
**Chino-shi Nagano, 391-0292 (JP)**

(72) Inventors:
• **HASHIMOTO Nobuaki**
**Chino-shi Nagano 391-0292 (JP)**
• **KOSUDA Tsukasa**
**Chino-shi Nagano 391-0292 (JP)**

(74) Representative: **Liesegang, Eva**
**Boehmert & Boehmert**
**Anwaltspartnerschaft mbB**
**Pettenkoferstrasse 22**
**80336 München (DE)**

(54) **PERSPIRATION AMOUNT MEASUREMENT DEVICE AND PERSPIRATION AMOUNT MEASUREMENT SYSTEM**

(57)    A perspiration amount measuring device 1 is mountable on an edge portion 102 of a helmet 100 and measures a perspiration amount from a head H. The perspiration amount measuring device 1 includes: an air flow path 100 through which inside air IA flows; a first thermo-hygro sensor 20 that is disposed at a place opened to an external field, and measures a temperature and a relative humidity of outside air OA that is taken in from a place on a side opposite to a wearer as viewed from the air flow path; a fan 30 that sucks the inside air IA and discharges the inside air IA to the outside; a second thermo-hygro sensor 40 that is disposed in a flow of the inside air IA generated due to an operation of the fan 30, and measures a temperature and a relative humidity of the inside air IA. The fan 30 discharges the inside air IA in a direction that intersects with a rotation axis AX about which a blade 33 rotates. The perspiration amount measuring device 1 can be popularly mounted on also a helmet for general use that is not equipped with a fan, and can measure a head perspiration amount more accurately compared to the prior art while having characteristics suitable for wearing on the head.

FIG. 1

## Description

Technical Field

The present invention relates to a perspiration amount measuring device and a perspiration amount measuring system.

Background Art

In a site of construction work or the like, there may be a case where workers have to work in an environment of high temperature. Under such a working condition, it is necessary to prevent the workers from suffering heat stroke. If a perspiration amount of a worker working under a high temperature environment, particularly a perspiration amount of the worker from his/her whole body (a whole body perspiration amount) can be grasped real time and a measure such as rehydration or stopping of the work is taken at a state prior to suffering from heat stroke, it is effective to prevent the workers from suffering from heat stroke. However, in general, to measure a perspiration amount from the workers whole body, it is considered that a large-sized facility becomes necessary. The measurement of a perspiration amount that requires such a large-sized facility is not suitable in a site of construction work or the like (see non-patent literature 1, for example).

To overcome such a drawback, a wearable perspiration amount measuring device has been studied by focusing on a helmet that a worker wears. Such a perspiration amount measuring device estimates a perspiration amount from the whole body by measuring a perspiration amount from a head in the helmet (see non-patent literature 2 and patent literature 1, for example).

The perspiration amount measuring devices described in non-patent literature 2 and patent literature 1 are configured such that air existing in a space that is formed between an outer shell of a helmet and a head of a wearer (a helmet inside air flow path) can be forcibly moved by a fan. On a premise of such a configuration, a moisture amount (inflow moisture amount $X_1$) per unit volume is calculated by measuring a temperature $t_1$ and a relative humidity $RH_1$ of air that flows into the helmet inside air flow path, and a moisture amount (outflow moisture amount $X_2$) per unit volume is calculated by measuring a temperature $t_2$ and a relative humidity $RH_2$ flown out from the helmet inside air flow path. A head perspiration amount equivalent amount (hereinafter simply referred to as a head perspiration amount) per unit time that is generated in the helmet inside air flow path can be obtained by subtracting the inflow moisture amount $X_1$ from the calculated outflow moisture amount $X_2$ and by multiplying an amount obtained by subtraction by an air volume F.

It has been found from studies that there exists a sufficient correlation between a head perspiration amount and a whole body perspiration amount (see non-patent literature 2) and hence, the whole body perspiration amount can be estimated based on a head perspiration amount obtained in the above-mentioned manner.

According to the perspiration amount measuring devices described in the non-patent literature 2 and the patent literature 1, a whole body perspiration amount can be estimated by measuring a head perspiration amount (equivalent amount) and hence, the perspiration amount measuring devices can contribute as measures to prevent a wearer from suffering from heat stroke without using a large-sized facility.

Citation List

Non-Patent Literature

Non-patent literature 1
Shinsuke Sawasaki and four others "Study on Thermal Evaluation Method for Hot Outdoor Environment" Seisan Kenkyu, 2006, volume 58, number 3, page 323 to 327
Non-patent literature 2: Tsukasa Kosuda and three others "Development of a Helmet Device Capable of Measuring Perspiration during Activity and Possibility of New Index for the Early Detection of Heat Stroke", Journal of Japan Institute of Electronics Packaging, General Incorporated Association "Japan Institute of Electronics Packaging", 2021, volume 24, number 6, page 541 to 550

Patent Literature

PTL 1: WO 2020/184686

Summary of Invention

Technical Problem

As described above, a wearable perspiration amount measuring device that uses a helmet estimates a whole body perspiration amount based on a measured value of a head perspiration amount and hence, it is important to measure the head perspiration amount as accurately as possible.

The techniques described in non-patent literature 2 and patent literature 1 are measuring methods that are established only with respect to a particular helmet that is equipped with a fan. However, in a site of construction work or the like, helmets that are manufactured by various manufacturers are used. Accordingly, the measurement of a perspiration amount on the premise of the particular helmet equipped with a fan is difficult to spread. In view of the above circumstances, there has been an expectation for the advent of a perspiration amount measuring device that can be popularly mounted also on a helmet for general use that is not equipped with a fan.

Further, a worker wears such a perspiration amount measuring device on his/her head and hence, the perspiration amount measuring device is expected to satisfy characteristics suitable for wearing on the head (characteristics suitable for wearable usages) such as, for example, a compact size, a light weight, power saving, low noise.

The present invention has been made in view of the above-mentioned circumstances, and it is an object of the present invention to provide a perspiration amount measuring device that can be popularly mounted on also a helmet for general use that is not equipped with a fan, and can measure a head perspiration amount more accurately compared to the prior art while possessing characteristics suitable for wearing on the head. It is another object of the present invention to provide a perspiration amount measuring system provided with such a perspiration amount measuring device.

Solution to Problem

According to an aspect of the present invention, there is provided a perspiration amount measuring device that is mountable on an edge portion of a helmet and is capable of measuring a perspiration amount from a head of a wearer of the helmet.

The perspiration amount measuring device includes: an air flow path through which "inside air" flows; a first thermo-hygro sensor that is disposed at a place opened to an external field, and measures a temperature and a relative humidity of "outside air" that is taken in from a place on a side opposite to the wearer as viewed from the air flow path; a fan that includes: a plurality of blades; and a fan case that houses the plurality of blades therein and is provided with a suction port and a discharge port, the plurality of blades being rotatable about a rotation axis so as to suck the inside air in the air flow path from the suction port and to discharge the inside air to an outside from the discharge port; and a second thermo-hygro sensor that is disposed in a flow of the inside air generated due to an operation of the fan, and measures a temperature and a relative humidity of the inside air. The fan discharges the inside air in a direction that intersects with the rotation axis. The "inside air" is air containing vapor generated from the head. The "outside air" is air in the external field of the perspiration amount measuring device. The meaning of the external field is described later.

According to another aspect of the present invention, there is provided a perspiration amount measuring system that includes: a helmet; and the above-described perspiration amount measuring device that is mounted on an edge portion of the helmet.

Advantageous effects of Invention

According to the perspiration amount measuring device of the present invention, it is possible to provide the perspiration amount measuring device that can be popularly mounted on also a helmet for general use that is not equipped with a fan, and can measure a head perspiration amount more accurately compared to the prior art while having characteristics suitable for wearing on the head. Further, according to the present invention, it is possible to provide a perspiration amount measuring system that includes such a perspiration amount measuring device.

Brief Description of Drawings

Fig. 1 is a cross-sectional view of a perspiration amount measuring device 1 and a perspiration amount measuring system 9 according to an embodiment 1.
Fig. 2 is a perspective view of the perspiration amount measuring device 1 illustrating the flow of inside air IA when a fan 30 is operated.
Fig. 3 is a cross-sectional view of the perspiration amount measuring device 1 illustrating the flow of the inside air IA when the fan 30 is operated.
Fig. 4 is a view illustrating an inner space 57 where a second thermo-hygro sensor according to the embodiment 1 is disposed.

Fig. 5 is a graph plotted by calculating an accumulated amount of a head perspiration amount equivalent amount by thermal fluid simulation while changing the level by making the place where the first thermo-hygro sensor 20 is disposed different.

Fig. 6 is a block diagram illustrating one example of electric hardware configuration implemented in the perspiration amount measuring device 1.

Fig. 7 is a perspective view illustrating an inside air collecting structure 80 according to an embodiment 2.

Fig. 8 is a view illustrating a function of the inside air collecting structure 80 according to the embodiment 2.

Fig. 9 is a view illustrating an inner space 57a where a second thermo-hygro sensor according to an embodiment 3 is disposed and an inside air collecting structure 80a according to the embodiment 3.

Fig. 10 is a view illustrating perspiration amount measuring devices 5, 5' according to an embodiment 5.

Fig. 11 is an essential part functional block diagram of an essential part of a perspiration amount measuring device 6 according to an embodiment 6.

Fig. 12 is a view illustrating a constitutional example of a salt concentration detection unit 310 according to the embodiment 6

Fig. 13 is a cross-sectional view illustrating an actual structure of an inner space 57b where a second thermo-hygro sensor according to a modification 1 is disposed.

Fig. 14 is a perspective view illustrating an actual structure of an inner space 57c where a second thermo-hygro sensor according to a modification 2 is disposed. Fig. 14 is an essential part perspective view corresponding to Fig. 7.

Fig. 15 is a view illustrating an actual structure of an inner space 57d where a second thermo-hygro sensor according to a modification 3 is disposed and an inside air collecting structure 80b.

Fig. 16 is a view illustrating sensor covers 50a, 50b, 50c, 50d according to a modification 4.

Fig. 17 is an essential part cross-sectional view illustrating a place where a second thermo-hygro sensor 40 according to a modification 5 is disposed.

Description of Embodiments

Hereinafter, the description is made with respect to a perspiration amount measuring device and a perspiration amount measuring system according to the present invention with reference to the drawings. With respect to the configurations and the structures that are shared in common in respective embodiments, the indication of symbols in the preceding drawing can be also used in the succeeding drawings and hence, there may be a case where the indication of symbols is omitted in the succeeding drawings. In this specification, with respect to the symbols shared by the respective embodiments, the contents that are described already using the symbols are applicable to the explanation of other drawings and hence, the description of the symbols in other drawings is omitted. Further, there is a case where "perspiration amount measuring device" is simply referred to as "device".

[Embodiment 1]

1. Configuration of perspiration amount measuring device 1 according to embodiment 1

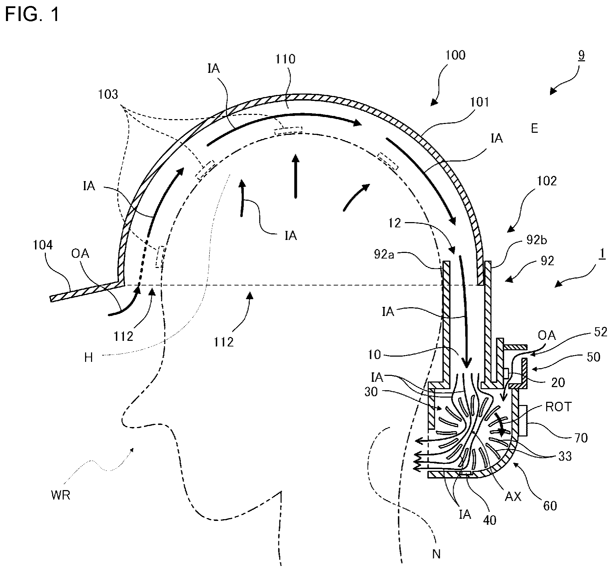

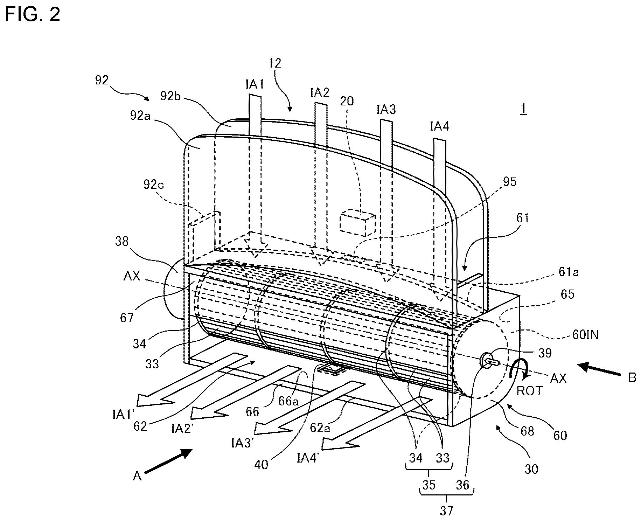

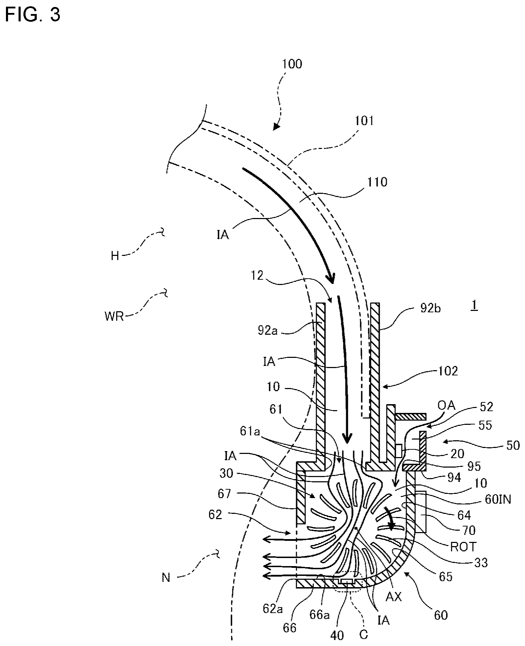

Fig. 1 is a cross-sectional view illustrating a perspiration amount measuring device 1 and a perspiration amount measuring system 9 according to an embodiment 1. With respect to the perspiration amount measuring device 1 illustrated in Fig. 1, the cross section of the perspiration amount measuring device 1 that is taken along a plane that is an imaginary plane perpendicular to a rotation axis AX, includes a first thermo-hygro sensor 20 and a second thermo-hygro sensor 40, and does not include an annular frame body 34 (described later), corresponds to a drawing as viewed along an arrow B in Fig. 2 (the same understanding being applied in Fig. 3, Fig. 8(c), Fig. 13, Fig. 15(b), Fig. 16(d) and Fig. 17). Fig. 2 is a perspective view of the perspiration amount measuring device 1 illustrating the flow of inside air IA when the fan 30 is operated. The illustration of a sensor cover 50, a control unit 70 and the like are omitted. Fig. 3 is a cross-sectional view of the perspiration amount measuring device 1 illustrating the flow of the inside air IA when the fan 30 is operated.

(1) Summary of perspiration amount measuring device 1

As illustrated in Fig. 1, the perspiration amount measuring device 1 according to the embodiment 1 is mounted on an edge portion 102 of a helmet 100. The perspiration amount measuring device 1 is a device that measures a perspiration amount from a head H of a wearer WR of the helmet 100.

The perspiration amount measuring device 1 includes a helmet mounting means (not indicated by a symbol) that is provided for mounting the perspiration amount measuring device 1 on the helmet 100. The perspiration amount measuring device 1 includes a clip 92, and the clip 92 forms a helmet mounting means. The clip 92 includes a clip inner peripheral member 92a, a clip outer peripheral member 92b, and a clip side peripheral member 92c (see also Fig. 2). An opening 12 is formed on one side (an upper side in the drawing) of the clip 92. When the edge portion 102 (a rear edge portion) of the helmet 100 engages with the opening 12 by fitting engagement, the edge portion 102 of the helmet 100 is fixed in a sandwiched manner between the clip inner peripheral member 92a and the clip outer peripheral member 92b. With such a configuration, the perspiration amount measuring device 1 is mounted on the helmet 100.

The perspiration amount measuring device 1 includes an air flow path 10, the first thermo-hygro sensor 20, the fan 30, and the second thermo-hygro sensor 40.

The perspiration amount measuring device 1 takes in air in an external field E (outside air OA) into the helmet 100 by rotating the fan 30, and makes air that contains vapor generated from the head H in the helmet 100 (inside air IA) flow, and discharges such air from the fan 30, and measures air that flows into such a space system and air that flows out from the space system by a first thermo-hygro sensor 20 and the second thermo-hygro sensor 40.

In such a configuration, in a broad definition, the "external field E" means a region outside a region as viewed from a region surrounded by an outer shell 101 (having a semispherical shape) of the helmet 100 and the perspiration amount measuring device 1 when the perspiration amount measuring device 1 is mounted on the helmet 100 and a human wears these elements. In other words, the "external field E" means a region on a side opposite to a wearer WR side with respect to the outer shell 101 of the helmet 100 or the perspiration amount measuring device 1. Further, in a narrow definition, the external field E may be also referred to as a region outside a space where the inside air IA (described later) flows.

[2] Air flow path 10

The air flow path 10 is a structural portion where air that contains vapor generated from the head H of the wearer WR (inside air IA) flows. The air flow path 10 is formed in a state where the air flow path 10 is surrounded by respective walls such as the clip inner peripheral member 92a, the clip outer peripheral member 92b, a clip-side peripheral member 92c, an inner wall 64 that form a fan case 60 described later and the like.

The air flow path 10 includes the opening 12 that is formed by one end of the clip inner peripheral member 92a and one end of the clip outer peripheral member 92b. An inner space that continuously extends from the opening 12 to the discharge port 62 of the fan case 60 forms the air flow path 10. The opening 12 is connected to a helmet inside air flow path 110 that is a space formed between the helmet 100 and the head H. The inside air IA can be taken into the air flow path 10 from such an opening 12.

(3) Thermo-hygro sensor 20

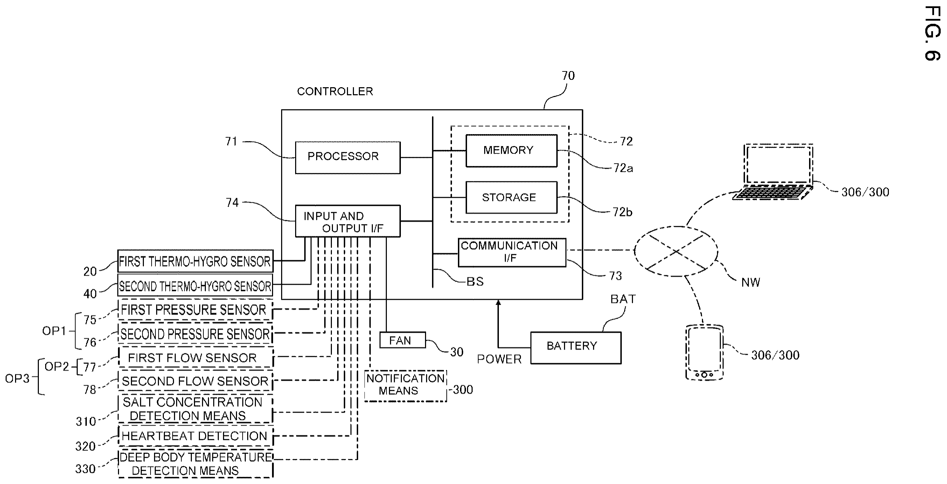

The first thermo-hygro sensor 20 is a sensor for measuring a moisture amount $X_1$ (also referred to as an absolute moisture $X_1$) contained per unit volume in the outside air OA that becomes air flowing into the air flow path 10. The first thermo-hygro sensor 20 is disposed at a place opened to the external field E, and measures a temperature and a relative humidity of outside air OA that is taken in from a place on a side opposite to a side of the wearer WR as viewed from the air flow path 10. The first thermo-hygro sensor 20 is electrically connected to a control unit 70 by a wire or a wireless (see Fig. 6), and transmits measured values obtained by the sensor to the control unit 70.

The first thermo-hygro sensor 20 is disposed in the place on the side opposite to the side of the wearer WR as viewed from the air flow path 10 through which the inside air IA flows. More specifically, the first thermo-hygro sensor 20 is disposed on a side opposite to a wearer WR side with reference to the clip outer peripheral member 92b.

(4) Sensor cover 50

The first thermo-hygro sensor 20 can be in a state where the first thermo-hygro sensor 20 is exposed to the external field E. In a case where the perspiration amount measuring device 1 is used indoor so that the sensor cover 50 is unnecessary, the structure of the perspiration amount measuring device 1 can be simplified by eliminating the cover thus reducing the weight of the perspiration amount measuring device 1.

However, it is preferable that the first thermo-hygro sensor 20 be further covered by the sensor cover 50 so as to make the first thermo-hygro sensor 20 minimally affected by a radiation heat from the outside such as solar beams particularly. Accordingly, the perspiration amount measuring device 1 includes the sensor cover 50 that covers the first thermo-hygro sensor 20.

An outside air intake port 52 is formed in the sensor cover 50 at a place on a side opposite to the wearer WR side as viewed from the air flow path 10 and also substantially above the first thermo-hygro sensor 20. The first thermo-hygro sensor 20 communicates with the external field E through the outside air intake port 52 and hence, it is safe to say that the first thermo-hygro sensor 20 is disposed at the place opened to the external field E. On the other hand, heat, moisture and the like generated from a wearer WR minimally enters such an outside air intake port 52. Accordingly, it is possible to take in the outside air OA that is in a state where the outside air is minimally affected by heat, moisture and the like generated from a wearer WR into the perspiration amount measuring device 1 and make the outside air OA impinge on the first thermo-hygro sensor 20.

As illustrated in Fig. 3, in the perspiration amount measuring device 1, a shield member 94 having a communicating portion 95 is disposed between the air flow path 10 and a space where the first thermo-hygro sensor 20 is disposed.

With such a configuration, when inside air IA in the air flow path 10 is sucked by the fan 30, outside air OA is forcibly sucked from the space where the first thermo-hygro sensor 20 is disposed through the communicating portion 95 along with such suction of inside air IA. As a result, in the space where the first thermo-hygro sensor 20 is disposed, the flow of outside air OA (the flow where outside air OA flows into from the outside air intake port 52, passes an area in the vicinity of the first thermo-hygro sensor 20, and reaches the communicating portion 95) is generated.

In this manner, according to the perspiration amount measuring device 1, the flow of outside air OA can be realized without providing a new fan, and the first thermo-hygro sensor 20 can be disposed in the space 55 where the flow of outside air OA is generated and hence, the perspiration amount measuring device 1 becomes a device that can perform the accurate measurement of a perspiration amount with high precision while realizing space saving, a compact size and the reduction of weight.

Sucked outside air OA merges with inside air IA in the vicinity of the communicating portion 95 in the air flow path 10. However, by setting a flow amount of merged outside air OA to an extremely small amount compared to inside air IA (an amount does not affect the function of the perspiration amount measuring device 1), it is possible to perform the practical perspiration amount measurement.

Further, discharging of inside air IA by the fan 30 (described later) is performed at a substantially constant air volume and hence, it is possible to allow outside air OA to flow in the space where the first thermo-hygro sensor is disposed (the space 55 where the flow of outside air OA is generated) at a substantially constant air volume. The space where the first thermo-hygro sensor 20 is disposed (the space 55 where the flow of outside air OA is generated) is formed such that outside air OA flows at a substantially constant air volume in the vicinity of the first thermo-hygro sensor 20.

In this manner, the configuration is adopted where outside air OA flows at a substantially constant air volume in the vicinity of the first thermo-hygro sensor 20. Accordingly, even if rapid and strong hot air or cool air is generated in the external field E, the first thermo-hygro sensor 20 can perform sensing of outside air OA in an environment where the first thermo-hygro sensor 20 is minimally affected by disturbance and hence, the accurate measurement of a head perspiration amount Y can be performed with high accuracy.

Further, in the perspiration amount measuring device 1, to focus on the flow of outside air OA, the first thermo-hygro sensor 20 is disposed upstream of the communicating portion 95 and in the vicinity of the communicating portion 95. By arranging the first thermo-hygro sensor 20 at such a position, it is possible to measure a temperature and a relative humidity in the vicinity of the communicating portion at which outside air OA is sucked and collected and hence, a measurement value of the head perspiration amount Y becomes more accurate.

Strictly speaking, although a slight amount of outside air OA is mixed around the second thermo-hygro sensor 40 (described later) via the communicating portion 95 described above, through the above-mentioned communicating portion 95, by setting a mixing amount of outside air OA to an extremely small amount, air that is an object to be measured by the second thermo-hygro sensor 40 is substantially regarded as inside air IA and hence, the measurement principle described later can be established in practical use.

(5) Fan 30

Next, returning to Fig. 1 to Fig. 3, the detail of the fan 30 is described.

The fan 30 includes: a plurality of blades 33; and a fan case 60 that houses the plurality of blade3 33 and is provided with a suction port 61 and a discharge port 62. The plurality of blades 33 rotate about a rotation axis AX so that inside air IA in the air flow path 10 is sucked from the suction port 61, and the inside air IA is discharged to the outside from the discharge port 62.

As the fan 30 according to the embodiment 1, a so-called cross flow fan can be adopted. As illustrated in Fig. 3, as viewed in a cross section of the fan 30 taken along a plane perpendicular to the rotation axis AX, the plurality of blades 33 are arranged at an equal interval along a circumferential direction about the rotation axis AX. The blades 33 are inclined at a predetermined angle with respect to the circumferential direction such that the blades 33 are inclined forward toward a front side in a rotational direction ROT.

On the other hand, when the fan 30 is viewed in a direction perpendicular to the rotation axis AX as indicated by an arrow A in Fig. 2, each blade 33 has an elongated lug shape. The respective blades 33 are fixed to at least two (five in the example illustrated in the drawing) annular frame bodies 34 such that one end side of each blade 33 is fixed to one annular frame body 34 and the other end side each blade 33 is fixed to the other annular frame body 34. The blades 33 and the annular frame bodies 34 constitute a bladed wheel impeller 35. Shafts 36 are connected to one end side and the other end side of the bladed wheel impeller 35 by way of the annular frame bodies 34. The shaft 36 disposed at one end side (at the left side in the drawing) is connected to a fan motor 38, and the shaft 36 disposed at the other end side (at the right side in the drawing) is received by a bearing 39.

A fan body 37 formed of the bladed wheel impeller 35 and the shaft 36 is housed in an inner space 60IN surrounded by the fan case 60 (a portion of the shaft being excluded). As described above, the fan case 60 is provided with the suction port 61 that faces the helmet inside air flow path 110 and the discharge port 62 that faces the external field E. On a fan case inner wall 64 extend from an edge portion 61a of the suction port to an edge portion 62a of the discharge port 62, the curved surface portion 65 having a curved shape along a circumference direction that the blades 33 rotate is formed. Further, a flow straightening portion 66a (symbol 66 indicating a flow straightening plate) is disposed within a range from the curved surface portion 65 to the edge portion 62a of the discharge port. The respective inner side surfaces of the side plate 68 and a shut plate 67 that is referred to as blanking plate also constitute the fan case inner wall 64.

The plurality of blades 33 are disposed adjacently to the air flow path 10 or in the air flow path 10.

The fan 30 (fan motor 38) is electrically connected to the control unit 70 (see Fig. 6 described later). The rotation of the fan 30 is controlled based on a drive signal or drive power supplied from the control unit 70. When the fan motor 38 is rotated, a rotational force of the motor is transmitted to the bladed wheel impeller 35 by way of the shaft 36 and hence, the blades 33 are rotated about the rotation axis AX. When the blades 33 are rotated in a clockwise direction, inside air IA is caught between two blades 33 disposed adjacently to each other (see Fig. 3), and the caught inside air IA flows to a position on a side opposite to a position where the inside air IA is caught by traversing a region in the vicinity of the rotation axis AX in the bladed wheel impeller 35 (cross flow), and the inside air IA is soon flows toward the discharge port 62 from between two another blades 33 and is discharged to the outside. Accordingly, the fan 30 is configured to discharge the inside air IA in the direction that intersects with the rotation axis AX (see an arrow indicated by IA or IAj in the respective drawings (j: natural number)).

When inside air IA is discharged to the outside, a negative pressure is generated in the air flow path 10 and the helmet inside air flow path 110. Accordingly, it is also safe to say that the fan 30 has a function of forcibly generating the flow of air existing in a space system constituted of the air flow path 10 and the helmet inside air flow path 110.

The position at which the fan 30 is disposed is set such that the destination of air generated by the fan 30 is set to a region ranging from a back of the head to a back of the neck N of a wearer WR in a state where the perspiration amount measuring device 1 is mounted on an edge portion 102 of the helmet 100. With such a configuration, air discharged from the fan 30 directly impinges on the region ranging from the back of the head to the back of the neck of the wearer WR and hence, air cools down the wearer WR or lowers a body temperature of the wearer WR thus positively contributing to prevent the wearer from suffering heat stroke.

(6) Second thermo-hygro sensor 40

The second thermo-hygro sensor 40 is a sensor for measuring a moisture amount $X_2$ (also refereed to as an absolute moisture $X_2$) contained per unit volume of inside air IA that is air discharged by the fan 30. The second thermo-hygro sensor 40 is disposed in the flow of inside air IA generated by operating the fan 30, and measures a temperature and a relative humidity of inside air IA. The second thermo-hygro sensor 40 is electrically connected with the control unit 70 by a wire or a wireless (see Fig. 6), and transmits a measured value obtained by sensing to the control unit 70.

The first thermo-hygro sensor 20 includes, besides a mode where a temperature sensor and a humidity sensor are integrally packaged, a mode where the temperature sensor and the humidity sensor are provided as separate bodies. Further, a first pressure sensor 75 according to an embodiment 4 described later may be integrally formed with a temperature sensor and/or a humidity sensor thus forming a package. In the same manner, also with respect to the second thermo-hygro sensor 40, the respective sensors that measures different physical quantities by sensing may be integrally formed with each other or may be formed as separate bodies.

(7) Arrangement of second thermo-hygro sensor 40

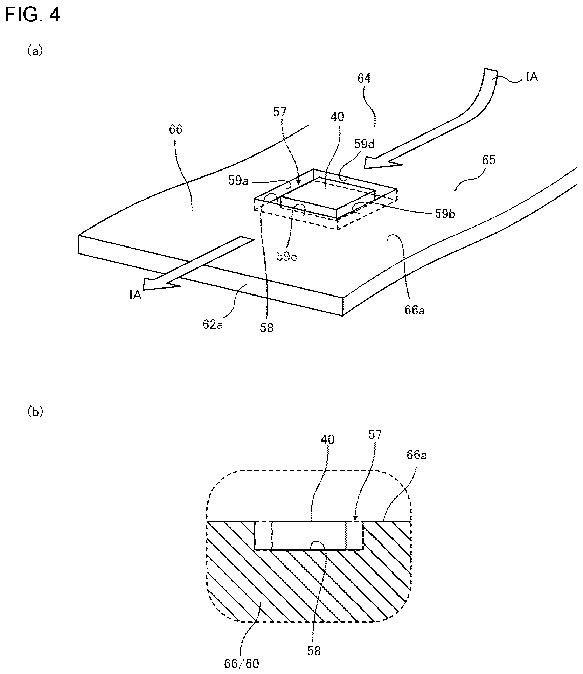

Fig. 4 is a view illustrating an inner space 57 where the second thermo-hygro sensor according to the embodiment 1 is disposed. Fig. 4(a) is an essential part perspective view illustrating only the region from an edge portion 62a of the discharge port of the fan case 60 to an intermediate portion of the flow straightening portion 66a. Fig. 4(b) is an enlarged cross-sectional view illustrating a region surrounded by a broken line C in Fig. 3 in an enlarged manner.

As illustrated in Fig. 4(a) and Fig. 4(b), the second thermo-hygro sensor 40 is disposed at a position on an upstream side of the inside air IA as viewed from the edge portion 62a of the discharge port. In other words, the second thermo-hygro sensor 40 is disposed at a slightly deep position as viewed from the discharge port 62 in the fan case 60 (see also Fig. 1 to Fig. 3).

In the embodiment 1, a recess that is indented from an inner side of the flow straightening plate 66 of the fan case 60 in a thickness direction is formed in the flow straightening plate 66. Such a recess is referred to as "an inner space 57 where the second thermo-hygro sensor 40 is disposed". The inner space 57 where the second thermo-hygro sensor 40 is disposed is defined from other portions by a sensor mounting surface 58 substantially perpendicular to the thickness direction of the fan case 60 and lateral walls 59 raised from the sensor mounting surface 58. The second thermo-hygro sensor 40 is disposed in a fitting engaging manner in the recess that is formed as the inner space 57 where the second thermo-hygro sensor is disposed.

As a result, with respect to the second thermo-hygro sensor 40, a bottom surface of the second thermo-hygro sensor 40 is brought into contact with the sensor mounting surface 58, side surfaces of the second thermo-hygro sensor 40 are surrounded by the lateral walls 59a to 59d on four sides such that side surfaces of the second thermo-hygro sensor 40 opposedly face the lateral walls 59a to 59d, and only an upper surface of the second thermo-hygro sensor 40 faces the inside of the fan case 60.

As illustrated in Fig. 4(a), it is preferable that the upper surface of the second thermo-hygro sensor 40 and the surface that constitutes the flow straightening portion 66a form coplanar surfaces (that is, be brought into a "coplanar" state).

(8) Measurement of head perspiration amount Y

The perspiration amount measuring device 1 measures basically a head perspiration amount Y using a principle of perspiration amount measurement described in non-patent literature 2. However, in the embodiment 1, the measurement of the head perspiration amount Y is not limited to such a principle.

Assuming a space formed between an outer shell 101 of the helmet 100 and the head H of the wearer WR as a helmet inside air flow path 110, the perspiration amount measuring device 1 measures a temperature $t_1$ and a relative humidity $RH_1$ of air in the external field E that flows into the helmet inside air flow path 110 or the outside air OA that is air equivalent to the air in the external field E using the first thermo-hygro sensor 20, and calculates a moisture amount per unit volume as an inflow moisture amount $X_1$. The perspiration amount measuring device 1 also measures a temperature $t_2$ and a relative humidity $RH_2$ of the inside air IA generated in the helmet inside air flow path 110 using the second thermo-hygro sensor 40, and calculates a moisture amount per unit volume as an outflow moisture amount $X_2$. Then perspiration amount measuring device 1 calculates the head perspiration amount Y that is a perspiration amount from the head H based on the inflow moisture amount $X_1$, the outflow moisture amount $X_2$, and an air volume F relating to discharging of the inside air IA by the fan 30. More specifically, the head perspiration amount Y is calculated by subtracting the inflow moisture amount $X_1$ from the outflow moisture amount $X_2$ and by multiplying a moisture amount obtained by the subtraction by the air volume F. Further, a whole body perspiration amount can be estimated based on the calculated head perspiration amount Y.

With the use of the measuring device that performs the measurement in accordance with such a principle, the manner of operation and advantageous effects of the perspiration amount measuring device 1 according to the embodiment 1 can be maximized. In other words, with use of the measuring device provided with the configuration of the perspiration amount measuring device 1 according to the embodiment 1, the measurement of the head perspiration amount and the whole body perspiration amount based on the above-mentioned principle can be performed more accurately. The detail relating to the calculation of the head perspiration amount Y is described in "example" described later.

2. Thermal fluid simulation example

The inventors of the present invention have obtained novel finding with respect to the arrangement position of the first thermo-hygro sensor 20 suitable for accurately measuring a head perspiration amount by performing thermal fluid simulation. This novel finding is described hereinafter.

(1) Thermal fluid simulation condition

It is assumed that a man wears a helmet provided with a helmet body and a fan that is mounted on the helmet body and discharges air in the helmet. It is also assumed that vapor and heat equivalent to vapor and heat that are generated when an average person wears the helmet are generated from the inside of the helmet. It is further assumed that a constant amount of perspiration per unit time is generated from a wearer WR (a person who wears the helmet). It is still further assumed that air flow having a speed of 1.5[m/s] impinges on the helmet from the back of the helmet.

It is assumed that inside air in the helmet inside air flow path or the air flow path is discharged by the fan. In such an operation, it is assumed that a temperature and a relative humidity of inside air are grasped by the second thermo-hygro sensor so that an outflow moisture amount is grasped. Further, it is assumed that a temperature and a relative humidity of air in the external field (outside air) that flows into the inside of the helmet are grasped by the first thermo-hygro sensor, so that an outflow moisture amount is grasped.

As a principle used for calculating a head perspiration amount (equivalent amount) Y, a principle of a perspiration amount measuring principle described in non-patent literature 2 is used.

It is assumed that the second thermo-hygro sensor is disposed inside the fan discharge port where discharging of the inside air by the fan is performed. The first thermo-hygro sensor is, at the first level (level 1), disposed in a place on a side opposite to a side of the wearer as viewed from the helmet inside air flow path or the air flow path. More specifically, it is assumed that the first thermo-hygro sensor is disposed outside the outer shell of the helmet. Further, the first thermo-hygro sensor is, at the second level (level 2), disposed near the inflow port of outside air in the inside of the helmet (see Fig. 1 in non-patent literature 2).

(2) Result and observation of thermal fluid simulation

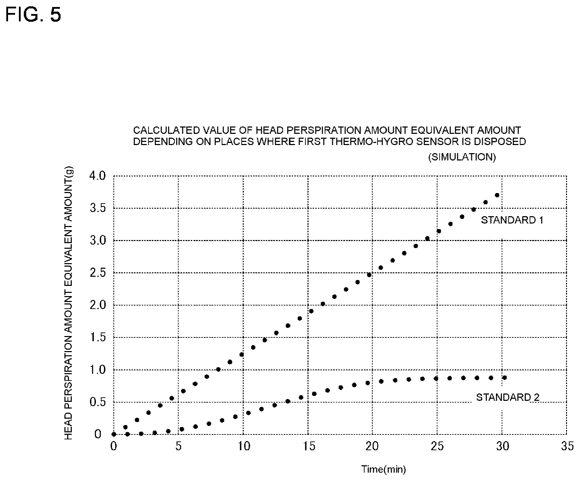

Fig. 5 is a graph plotted by calculating an accumulated amount of a head perspiration amount equivalent amount by simulation while changing the level by making the place where the first thermo-hygro sensor 20 is disposed different.

As indicated by the graph illustrated in Fig. 5, at the level 1, the head perspiration amount equivalent amount (accumulated amount) was increased linearly with the lapse of time. On the other hand, at the level 2, although the tendency that the head perspiration amount equivalent amount (accumulated value) is increased along with the lapse of time, the accumulated value was saturated when a certain time period has elapsed.

In a case where a constant amount of perspiration per unit time is produced from a wearer WR, it is considered that an actual head perspiration amount (accumulated value) was also linearly increased with the lapse of time naturally. To observe the present thermal fluid simulation from this point, the graph was not linearly increased at the level 2. This is considered that, although the arrangement position of the first thermo-hygro sensor at the level 2 was the position at which air in the external field was taken in, the position was the position where a possibility exists that heat and moisture generated from a neck, a forehead and the like of the wearer were also mixed so that the accuracy of the measurement of the head perspiration amount equivalent amount was decreased.

On the other hand, with respect to the graph at the level 1, the head perspiration amount equivalent amount (accumulated value) was increased linearly with the lapse of time, and it was found that the value close to the actual head perspiration amount was obtained when the arrangement position of the first thermo-hygro sensor at the level 1 was adopted. From the above-mentioned result of the thermal fluid simulation, in constituting the perspiration amount measuring device 1, it was confirmed that it is preferable to arrange the first thermo-hygro sensor at the place on a side opposite to a side of a wearer as viewed from the helmet inside air flow path or the air flow path in accordance with the level 1.

3. Embodiment

Next, an embodiment of the perspiration amount measuring device 1 is described. However, the description made hereinafter is an example by all means, and the present invention is not limited to the contents described hereinafter.

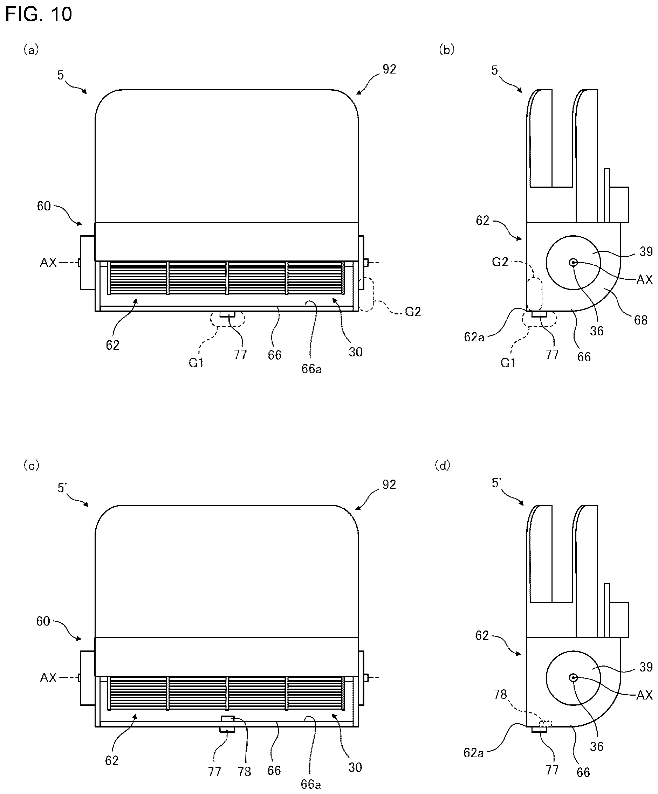

The clip inner peripheral member 92a and the clip outer peripheral member 92b of the perspiration amount measuring device 1 are curved tracing a curved shape of an edge portion 102 of the helmet (for example, see Fig. 10(b), Fig. 10(d) used in the description of an embodiment 5 described later). In the perspiration amount measuring device 1, the control unit 70 that includes a battery (not indicated by a symbol) is disposed at a position on a back side of the fan case 60.

Fig. 6 is a block diagram illustrating one example of electric hardware configuration implemented in the perspiration amount measuring device 1. As illustrated in Fig. 6, the perspiration amount measuring device 1 according to the embodiment is mainly constituted of the control unit 70 electrically.

The control unit 70 is a computer, and includes a processor 71, a memory 72a and a storage 72b (collectively referred to as a memory part 72), a communication I/F (interface) 73, and an input/output I/F (interface) 74. These are connected to a bus BS. The battery BAT supplies electricity to the control unit 70. The communication I/F may include a wireless communication means.

The processor 71 is operated in accordance with a program stored in the memory part 72, and performs a control of the respective units. The memory part 72 also includes a non-volatile memory device (ROM or the like). The memory part 72 stores: a boot program executed by the processor 71 at the time of starting the perspiration amount measuring device 2; and a program dependent on a hardware of the control unit 70 and the like.

The communication I/F 73 performs the transmission and the reception of data with an external management device (not illustrated in the drawing) via a wireless communication unit (not indicated by a symbol). The input/output I/F 73 functions as a go-between between the control unit 70 and an information terminal device 306 (so-called smart phone, PC or the like) via a network NW, for example. The input/output I/F 74 is electrically connected to the first thermo-hygro sensor 20, the second thermo-hygro sensor 40, the fan 30 and the like respectively, and is operated as an interface with these input/output devices.

The processor 71 performs a rotation control of the fan 30. The processor 71 calculates a head perspiration amount Y based on information from the first thermo-hygro sensor 20 and the second thermo-hygro sensor 40 via the input/output I/F 74. Further, the processor may calculate a whole body perspiration amount based on the calculated head perspiration amount Y. The processor 71 controls the data transmission/reception between the processor 71 and an external management device (not illustrated in the drawing) via the communication I/F 73. The processor 71 can be operated so as to hold data relating to the calculated head perspiration amount Y or the calculated whole body perspiration amount in the memory part 72.

The control unit 70 calculates a moisture amount per unit volume as an inflow moisture amount $X_1$ based on a temperature $t_1$ and a relative humidity $RH_1$ of outside air OA received from the first thermo-hygro sensor 20.

Various approximations are available for estimating a moisture amount (absolute humidity) per unit volume from relative humidity. In this example, the estimation is made using the relatively well-known Tetens formula.

Assuming the inflow moisture amount as $X_1$ [g/m$^3$], a temperature of the outside air OA as $t_1$[°C], a relative humidity of the outside air OA as $RH_1$ [%], and a saturated vapor pressure of the outside air OA as $e_1$ [hPa], the control unit 70 obtains the inflow moisture amount $X_1$ by performing calculations using the following formulae (1) and (2).

In a case where the formula is applied by picking up a subscript 1 in any one of respective terms e, t, RH, X in the formulae (1) to (3), it is assumed that the subscript 1 is also picked up and is applied in other terms. In the same manner, in a case where the formula is applied by picking up a subscript 2 in any one of respective terms e, t, RH, X in the formulae (1) to (3), it is assumed that the subscript 2 is also picked up and is applied in other terms.

$$e_{1,2}(hPa) = 6.11 \times 10^{(7.5t_{1,2})/(t_{1,2} + 273.3))} \dots (1)$$

$$X_{1,2}(g/m^3) = (217^*e_{1,2}/(273.15+t_{1,2})) \times (RH_{1,2}/100) \dots (2)$$

In the same manner, the control unit 70 calculates a moisture amount per unit volume as an outflow moisture amount $X_2$ based on a temperature $t_2$ [°C] and a relative humidity $RH_2$[%] of inside air IA received from the second thermo-hygro sensor 40. More specifically, assuming the outflow moisture amount as $X_2$ [g/m$^3$], a temperature of the inside air IA as $t_2$[°C], a relative humidity of the inside air IA as $RH_2$ [%], and a saturated vapor pressure of the inside air IA as $e_2$ [hPa], the control unit 70 obtains the outflow moisture amount $X_2$ by performing calculations using the above-mentioned formulae (1) and (2).

The control unit 70 obtains the head perspiration amount Y (g/m$^3$) by performing calculation by the following formula (3) based on the inflow moisture amount $X_1$ [g/m$^3$] and the outflow moisture amount $X_2$ [g/m$^3$] obtained by the above-mentioned calculation and a given (known) air volume F [m$^3$/min] of the fan 30.

$$Y(g/min) = (X_2 - X_1) \times F(m^3/min) \dots (3)$$

Further, the control unit 70 can also estimate the whole body perspiration amount based on the obtained head perspiration amount Y.

The control unit 70 includes "an inflow moisture amount calculation part 214 (not illustrated in the drawing)" that calculates a moisture amount per unit volume as the above-mentioned inflow moisture amount $X_1$, "an outflow moisture amount calculation part 216 (not illustrated in the drawing)" that calculates a moisture amount per unit volume as the outflow moisture amount $X_2$, and "a head perspiration amount calculation part 218" that calculates a head perspiration amount Y based on the inflow moisture amount $X_1$, the outflow moisture amount $X_2$, and the air volume F relating to discharging of the inside air IA by the fan 30. Further, the control unit may include "a whole body perspiration amount calculation part 210" that estimates a whole body perspiration amount based on the calculated head perspiration amount Y.

The control unit 70 disposed in the perspiration amount measuring device may include all of the inflow moisture amount calculation part, the outflow moisture amount calculation part, the head perspiration amount calculation part and the whole body perspiration amount calculation part. Alternatively, the management device disposed outside the perspiration amount measuring device may include some of or all of the inflow moisture amount calculation part, the outflow moisture amount calculation part, the head perspiration amount calculation part and the whole body perspiration amount calculation part.

4. Advantageous effects of perspiration amount measuring device 1 according to the embodiment 1

(1-1) In the perspiration amount measuring device described in non-patent literature 2, the thermo-hygro sensor (corresponding to the first thermo-hygro sensor 20) on an inflow air side is disposed near an inner periphery of the helmet. Although such a sensor arrangement position is the position at which air in the external field is taken in, there is a possibility that heat and moisture generated from a neck, a forehead, a breast, a back and the like of a wearer are mixed into air from the external field at such a position. In view of the above, with respect to the configuration of the perspiration amount measuring device described in non-patent literature 2, there is a possibility that the measurement of the heat perspiration amount is affected by the heat and the moisture from the wearer and hence, there is still room for improvement in the more accurate measurement of the head perspiration amount.

On the other hand, according to the perspiration amount measuring device 1 of the embodiment 1, the first thermo-hygro sensor 20 is disposed at the place released (opened) to the external field E and hence, the first thermo-hygro sensor 20 measures a temperature and a relative humidity of air in the external field E sucked from the place on a side opposite to a side of the wearer WR as viewed from the air flow path 10. Accordingly, near the first thermo-hygro sensor 20, fresh outside air is taken in in a state where the mixing of the heat and the moisture from heat and moisture generated from a head, a neck, a forehead, a breast, a back and the like of the wearer WR is suppressed. The first thermo-hygro sensor 20 senses such air and hence, an affect of the heat and the moisture generated from the wearer WR can be suppressed whereby the head perspiration amount Y can be measured more accurately.

(1-2) Further, the perspiration amount measuring device 1 is configured such that the plurality of blades 33 rotate about the rotation axis AX so that the inside air IA in the air flow path 10 is sucked from the suction port 61, and the inside air IA is discharged to the outside from the discharge port 62. With such a configuration, it is possible to allow the most of the inside air IA generated by the head H of the wearer WR in the helmet to flow by the fan 30. The second thermo-hygro sensor 40 is disposed in the flow of the inside air IA that is generated by operating the fan 30. With such a configuration, a temperature $t_2$ and a relative humidity $RH_2$ of the inside air IA can be properly detected by sensing and hence, a head perspiration amount Y can be measured more accurately.

(1-3) In a case where an axial type fan (a fan that discharges inside air along a rotation axis but not in a direction that intersects with the rotation axis (see the inventions that are described in Japanese Patent Application No. 2022-64656, PCT/JP2023/3297 and were made by the inventors of the present invention)) is used as the fan 30, for example, the inside air IA that flows downward on a back of the head side of the helmet inside air flow path 110 and substantially parallel to such a back of the head side has its flow direction forcibly bent at an acute angle in the vicinity of a suction port of the fan and, thereafter, is forcibly stirred by a propeller-type blade and is discharged to the outside. In this manner, in the perspiration amount measuring device that uses the axial type fan, a flow path resistance of the inside air IA becomes large as a whole and hence, air supply efficiency per unit electric power cannot be increased so much. Further, noises such as wind noises generated by the blade and operation noises of a fan motor are become large.

On the other hand, as described in the embodiment 1, in a case where the fan such as a cross-flow fan, for example, where inside air IA is discharged in the direction that intersects with the rotation axis AX is used as the fan 30, as illustrated in Fig. 2, inside airs IA1, IA2, IA3, IA4 that flow downward on a back of the head side of the helmet inside air flow path 110 and substantially parallel to such a back of the head side are discharged from the discharge port 62 as indicated by IA1', IA2', IA3', IA4' by being guided by the movement of the blade 33 without being stirred and maintaining the parallel relationship. In this case, the inside air IA smoothly flows while maintaining the parallel relationship from an upstream side to a downstream side (a discharge port 62 side) of the fan 30.

In this manner, the perspiration amount measuring device 1 that uses the fan 30 according to the embodiment 1 does not generate the stirring that is generated by a propeller type blade such as an axial type fan. Accordingly, the flow path resistance of the inside air IA becomes small as a whole and hence, air supply efficiency per unit electric power becomes high (high air supply efficiency).

In the embodiment 1, the air supply efficiency is high and hence, even when a rotational speed of the fan is not so much high, a required air volume can be ensured. Further, noises such as wind noises generated by the blade 33 and operation noises of a fan motor 38 do not become large and hence, feeling of noise can be reduced (low noise). The perspiration amount measuring device 1 that uses such a fan 30 is considerably suitable as a wearable device that is mounted near an ear.

Further, the fan 30 according to the embodiment 1 exhibits high air supply efficiency and hence, the consumption of electric power for securing a required air volume becomes small (low electric power consumption) compared to an axial fan.

The fan 30 according to the embodiment 1 consumes low electric power and hence, for example, when the fan is operated using the same battery, the fan 30 according to the embodiment 1 can be operated longer until the battery runs out compared to a case of an axial type fan. On the other hand, in a case where the same operation time is to be secured, the fan 30 according to the embodiment 1 consumes small amount of electric power compared to the axial type fan and hence, eventually, it is sufficient for the fan according to the embodiment 1 to use a small-sized and light-weighted battery. Further, in case of the axial type fan where air supply efficiency is not so high, in an attempt to increase a size of the blade for increasing an air volume, not only a diameter of the fan but also the size of a fan motor, a fan case and the like also become large and hence, the entirety of the device becomes large-sized and heavy. The fan 30 according to the embodiment 1 originally has high air supply efficiency and hence, it is unnecessary to increase the sizes of the blade whereby the fan 30 according to the embodiment 1 can maintain a small sized and light-weighted configuration (small size, light weight).

Assume a case where a perspiration amount measuring device is constituted using an axial type fan having a relatively large size and is heavy, and such a device is mounted on the edge portion 102 of the helmet 100, the center of gravity of the fan is located at the position remote from the center portion of the helmet and the fan itself is heavy and hence, when the wearer WR bends only his/her head H downward by looking down and returns the head H to an original posture, a large moment is generated due to the weight of the fan so that the wearer WR feels a fatigue on his/her neck.

On the other hand, the fan 30 according to the embodiment 1 is small sized and light in weight and hence, the above-mentioned moment can be reduced whereby the wearer WR can reduce a fatigue on his/her neck.

As has been described above, by adopting the fan that discharges the inside air IA in the direction that intersects with the rotation axis AX as the fan 30, the perspiration amount measuring device 1 can expect high air supply efficiency, low noise, low power consumption, and the small-sized and light weight configuration and hence, the perspiration amount measuring device 1 becomes a perspiration amount measuring device that includes characteristics suitable for mounting on the head.

(1-4) Due to the above-mentioned configuration, the perspiration amount measuring device 1 according to the embodiment 1 can be popularly mounted on the general type helmet equipped with no fan, and more accurately measure a head perspiration amount compared to the prior art while possessing characteristics suitable for mounting on the head. Further, these accurate measurements can be achieved with the relatively simple configuration without increasing the number of parts such as the number of fans and the number of sensors.

(2) In the perspiration amount measuring device described in the non-patent literature 2, the fan is formed in a state where the fan is embedded in a portion of the outer shell of the dedicated helmet (helmet equipped with the fan) and hence, there exists a circumstance that a so-called large fan cannot be introduced. Accordingly, in the prior art, in a case where the wearer WR generates a large amount of perspiration during a short time, discharging of air by the fan cannot catch up with the generation of perspiration and hence, there arises a drawback that the measurement of a perspiration amount cannot follow a change in a perspiration generation state whereby there is still room for improvement in terms of the more accurate and precise measurement of a perspiration amount.

On the other hand, in the perspiration amount measuring device 1 according to the embodiment 1, the perspiration amount measuring device 1 measures a perspiration amount in a state where the perspiration amount measuring device 1 is mounted on a commercially available helmet externally. Naturally, the fan 30 is also mounted on the helmet body externally and hence, the degree of freedom in selecting the fan is increased. The perspiration amount measuring device 1 according to the embodiment 1 can adopt a fan that is large-sized and has a large air volume and a high efficiency compared to a fan that is incorporated in the helmet. By suitably adopting such a fan having high performance, even if the wearer WR generates a large amount of perspiration during a short time, the fan can discharge the inside air IA with sufficient ability and hence, for example, it is possible to perform the measurement that speedily follows the sharp increase of a perspiration amount. Also from this point of view, the perspiration amount measuring device can expect the measurement with high accuracy and high precision.

(3) For example, the fan 30 that discharges the inside air IA in the direction that intersects with the rotation axis AX as in the case of the cross flow type fan, the fan 30 has the discharge port 62 having a wide opening area in general and hence, the fan 30 is in a state where the outside air OA flows in a reverse direction.

In the perspiration amount measuring device 1, the configuration is adopted where the second thermo-hygro sensor 40 is disposed at the position on an upstream side of the inside air IA as viewed from the edge portion 62a of the discharge port (the configuration where the second thermo-hygro sensor 40 is disposed on a deep side as viewed from the discharge port 62). Accordingly, even if the outside air OA slightly enters the perspiration amount measuring device 1 from the discharge port 62, the entered outside air OA is pushed back by a main stream of the inside air IA and hence, the outside air OA does not reach the position where the second thermo-hygro sensor 40 is disposed whereby the second thermo-hygro sensor 40 is not affected by the outside air OA (the affect of the outside air OA to the second thermo-hygro sensor 40 being reduced). In this manner, the perspiration amount measuring device 1 has the structure where the perspiration amount measuring device 1 is minimally affected by disturbance of the outside air OA blowing into the perspiration amount measuring device 1 from the discharge port 62 and hence, a head perspiration amount can be more accurately measured.

(4) In the perspiration amount measuring device 1, the inner space 57 in which the second thermo-hygro sensor is disposed is defined by the sensor mounting surface 58 substantially perpendicular to the thickness direction of the fan case 60 and the lateral walls 59 that are raised from the sensor mounting surface 58.

With such a configuration, there is no possibility that the outside air OA enters the inner space 57 in a state where the outside air OA traverses at least the sensor mounting surface 58 and the lateral walls 59. The second thermo-hygro sensor 40 is disposed in an environment that is surrounded by the sensor mounting surface 58 and the lateral walls 59 so that outside air OA minimally intrudes into the environment and hence, the perspiration amount measuring device 1 is minimally affected by the disturbance of the outside air OA blown into the perspiration amount measuring device 1 from the discharge port 62 whereby a head perspiration amount can be measured more accurately.

Particularly, in the perspiration amount measuring device 1 illustrated in Fig. 1 to Fig. 4 as an example of the embodiment 1, side surfaces of the second thermo-hygro sensor 40 are surrounded by the lateral walls 59a to 59d on four sides in an oppositely facing manner, and only an upper surface of the second thermo-hygro sensor 40 faces the inside of the fan case 60 (where a large amount of inside air IA is flowing) and hence, the outside air OA is particularly minimally mixed into the inside air IA.

Further, the upper surface of the second thermo-hygro sensor 40 and a surface that constitutes the flow straightening portion 66a are in a so-called coplanar state. Accordingly, the inside air IA that flows on the surface of the flow straightening portion 66a passes the same plane having substantially no step and hence, the flow path resistance is suppressed whereby an energy loss and the generation of noise caused by the flow path resistance can be suppressed.

(5) The perspiration amount measuring device 1 according to the embodiment 1 includes the sensor cover 50 that covers the first thermo-hygro sensor 20. Since the perspiration amount measuring device 1 includes such a cover, in the same manner as a ventilated case for meteorological instruments used for weather observation, it is possible to prevent a radiation heat from sun, a ground, buildings and the like, a radiation heat, a convection heat and the like from a wearer, a moisture (vapor) that the wearer generates and the like from being directly transferred to the first thermo-hygro sensor 20. Accordingly, the first thermo-hygro sensor 20 can perform a sensing operation while suppressing direct influence from these heats, moisture and the like and hence, the head perspiration amount Y can be more accurately measured.

(6) The first thermo-hygro sensor 20 according to the embodiment 1 is disposed in the space where the flow of the outside air OA is generated. By arranging the first thermo-hygro sensor 20 in the space where the flow of the outside air OA exists besides simply bringing the first thermo-hygro sensor 20 into contact with outside air OA, the fresh outside air OA that is not affected by heat generated by the perspiration from a human body constantly impinges on the first thermo-hygro sensor 20. Accordingly, the head perspiration amount Y can be measured more accurately.

5. Perspiration amount measuring system 9 according to the embodiment 1

Returning to Fig. 1, a perspiration amount measuring system 9 is described.

The perspiration amount measuring system 9 can be formed by combining the perspiration amount measuring device 1 and the helmet 100. The perspiration amount measuring system 9 includes: the helmet 100; and the perspiration amount measuring device 1 according to the embodiment 1 mounted on the edge portion 102 of the helmet 100.

As the helmet 100, a commercially available general-use helmet can be adopted. For example, a work-use helmet used in general in construction work and the like can be named.

The helmet 100 includes an outer shell 101, an intermediate covering member 103, a brim 104 and the like. The outer shell 101 is a member that makes the external field E and the head H of the wearer WR spaced apart from each other, and protects the head H from the external field E. A helmet inside air flow path 110 is formed between the outer shell 101 and the head H of the wearer WR. The intermediate covering member 103 is, for example, an inner belt or the like, and supports the helmet 100 by bringing the helmet 100 into contact with the head. The intermediate covering member 103 has an opening, and vapor and hot air move back and forth between the head H and the helmet inside air flow path 110.

As the perspiration amount measuring device, the perspiration amount measuring device 1 according to the embodiment 1 is used and hence, the perspiration amount measuring system 9 acquire substantially the same advantageous effect as the advantageous effects that the perspiration amount measuring device 1 according to the embodiment 1 possesses. With the use of such perspiration amount measuring system 9, a head perspiration amount can be measured more accurately, and, further, it is possible to take a proper measure against a heat stroke.

In the above-mentioned configuration, as the example, a mode where the perspiration amount measuring system 9 is constituted by using the perspiration amount measuring device 1 according to the embodiment 1 is described. However, the perspiration amount measuring system is not limited to the perspiration amount measuring system 9. That is, the perspiration amount measuring system can be also constituted using perspiration amount measuring devices according to the respective embodiments and perspiration amount measuring devices according to respective modifications described later.

[Embodiment 2]

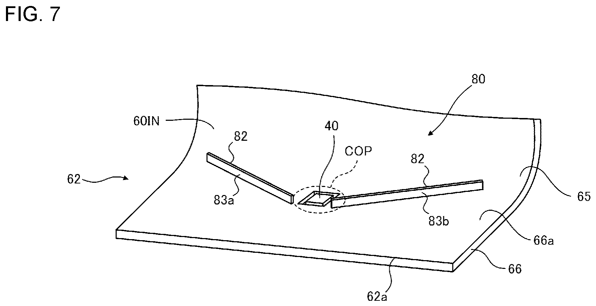

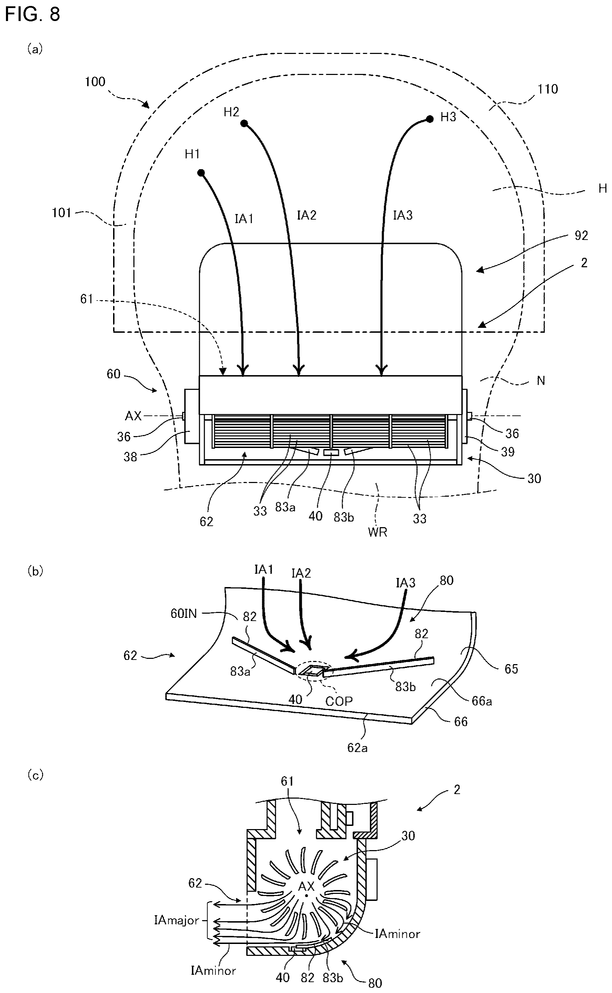

Fig. 7 is a perspective view describing an inside air collecting structure 80 according to the embodiment 2. Fig. 7 is a view corresponding to Fig. 4(a), and is a perspective view of an essential part illustrating a region from an edge portion 62a of a discharge port of a fan case 60 to an intermediate portion of a flow straightening portion 66a. Fig. 8 is a view illustrating a function of the inside air collecting structure 80 according to the embodiment 2. Fig. 8(a) is a front view of the perspiration amount measuring device 1 as viewed from a discharge port 62 side. A helmet 100 on which the perspiration amount measuring device 1 is mounted and a head H of a wearer WR are indicated by a phantom line. Fig. 8(b) is a perspective view corresponding to Fig. 7, and Fig. 8(c) is a cross-sectional view corresponding to Fig. 3.

The perspiration amount measuring device 2 according to the embodiment 2 has basically substantially the same configuration as the perspiration amount measuring device 1 according to the embodiment 1. However, the perspiration amount measuring device 2 differs from the perspiration amount measuring device 1 according to the embodiment 1 with respect to a point (a different point) that the perspiration amount measuring system 2 includes the inside air collecting structure 80. Hereinafter, the perspiration amount measuring system 2 is described by focusing on the different point.

In the perspiration amount measuring system 2 according to the embodiment 2, at a position inside the fan case 60 and near the discharge port 62, the inside air collecting structure 80 is disposed. The inside air collecting structure 80 is configured such that, due to the provision of an air guide that guides the flow of air, with respect to some inside airs $IA_{minor}$ out of inside airs (a total sum of $IA_{major}$ and $IA_{minor}$) to be discharged, the inside airs $IA_{minor}$ that flow in parallel to each other are mixed and are merged together as the inside airs $IA_{minor}$ move toward the discharge port 62 (see Fig. 7 and Fig. 8).

In the example illustrated in Fig. 7 and Fig. 8, as the inside air collecting structure 80, two ridge-like ribs 83a, 83c are disposed in the flow straightening portion 66a.

Out of walls that the ribs 83a, 83c have, walls on an upstream side of inside airs IA form guide walls 82 that function as air guides. Surfaces of the the guide walls 82 intersect with the direction that air flow of the inside airs IA enter from upstream of the inside air collecting structure 80. Accordingly, when the inside airs IA flow from an upstream side, some inside airs IA impinges on the surfaces of the guide walls 82 at certain angles and are guided to a predefined direction. The surfaces of two guide walls 82 that belong to the ribs 83a, 83c are configured to approach to each other as the surfaces advance to the edge portion 62a of the discharge port, and the surfaces of the guide walls 82 are converged toward a collecting place COP on the inside airs. In such a configuration, a second thermo-hygro sensor 40 is disposed in the vicinity of the place COP where the inside airs in the inside air collecting structure 80 are collected.

Using a surface of the flow straightening portion 66a as a reference for determining a height of the guide walls 82 that belong to the ribs 83a, 83c, the height of the guide walls 82 is set sufficiently small with respect to a height of the discharge port 62.

It is considered that irregularities exist with respect to the distribution of blood vessels and the distribution of sweat glands in a head of a human. Accordingly, to compare a calorific value and a perspiration amount among different places $H_1$, $H_2$, $H_3$ of the head H, the specific place may have a large/small calorific value or a large/small perspiration amount compared to other places. Also with respect to temperatures and humidities of the inside airs IA1, IA2, IA3 ... generated from the respective places $H_1$, H2, $H_3$ ..., there is a possibility that the temperatures and humidities are different values and distributions.

On the other hand, as illustrated in Fig. 8(a)(also see Fig. 2), the fan 30 and the fan case 60 of the perspiration amount measuring device 2 (substantially the same with respect to the perspiration amount measuring device 1), originally, have the specification where an air flow that includes the inside airs IA1, IA2, IA3 corresponding to the respective portions generated from a plurality of arbitrary portions $H_1$, H2, H3 ... of the head H of the wearer WR is sucked into the inside of the fan case 60 from the suction port 61 while allowing the inside airs to maintain the parallel relationship with each other and is discharged from the discharge port 62 while allowing the inside airs to maintain the parallel relationship with each other (the parallel relationship described above indicating a mode as viewed from a direction perpendicular to a rotation axis AX).

Accordingly, depending on the position downstream of the inside airs IA at which the second thermo-hygro sensor 40 is arranged, the value of temperature and the value of humidity obtained from the sensor differ and hence, there is a possibility that the temperature and the humidity that represent the whole head H of the wearer WR cannot be detected by sensing.

The perspiration amount measuring device 2 according to the embodiment 2 includes the above-mentioned inside air collecting structure 80, and as illustrated in Fig. 8(b), the inside airs IA1, IA2, IA3 ... that flow from an upstream side are mixed with each other while advancing to the place COP where the inside airs are collected by being guided by the guide walls 82, and are collected to the inside air having a temperature and a humidity that represent the whole head H of the wearer WR. The second thermo-hygro sensor 40 is disposed in the vicinity of the place COP where the inside airs are collected, and detects the inside air IA by sensing based on the inside air that represent the whole head H and hence, the perspiration amount measuring device 2 can perform the more accurate head perspiration amount measurement.

In the perspiration amount measuring device 2 according to the embodiment 2, the height of the guide walls 82 of the inside air collecting structure 80 is set sufficiently small compared to the height of the discharge port 62. Accordingly, among the total inside air (the total sum of the $IA_{major}$ and $IA_{minor}$) that flows toward the discharge port 62, a volume rate of the $IA_{minor}$ relating to the mixing and merging of the inside airs IA1, IA2, IA3 can be made extremely small compared to the entire volume. Accordingly, the inside airs $IA_{major}$ that are the major portion of the inside airs that flow above the guide walls 82 (using the surface of the flow straightening portion 66a as the reference height) flow straightly and are discharged without being influenced by the inside air collecting structure 80 and hence, practically, the increase of the flow path resistance of the inside air IA can be almost avoided. Accordingly, the perspiration amount measuring device 2 according to the embodiment 2, while aiming at the acquisition of accuracy in the measurement of the head perspiration amount, at the same time, can maintain characteristics such as high air supply efficiency, small noise and saving of power.

In this embodiment, the configurations of the perspiration amount measuring device of this embodiment are basically substantially equal to the configurations of the perspiration amount measuring device of the preceding embodiment except for the point that the inside air collecting structure 80 is provided. Accordingly, this embodiment can also acquire the advantageous effects based on the configurations shared in common by these embodiments in the same manner.

[Embodiment 3]

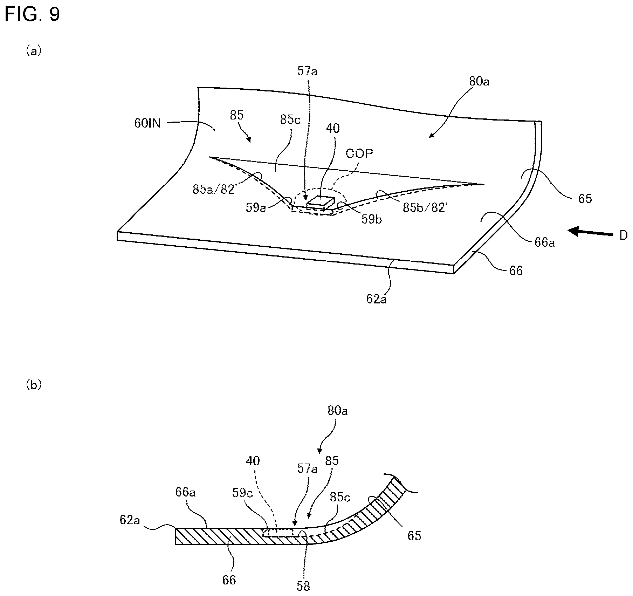

Fig. 9 is a view illustrating an inner space 57a where a second thermo-hygro sensor according to an embodiment 3 is disposed and an inside air collecting structure 80a according to the embodiment 3. Fig. 9(a) is a perspective view of an essential part corresponding to Fig. 7. Fig. 9(b) is a right side view when the inner space 57a in which the second thermo-hygro sensor is disposed and the inside air collecting structure 80a are viewed along an arrow in Fig. 9(a).

The perspiration amount measuring device (not indicated by a symbol) according to the embodiment 3 has basically substantially the same configuration as the perspiration amount measuring devices (perspiration amount measuring devices 1, 2) according the above-mentioned preceding embodiments. However, the perspiration amount measuring device according to the embodiment 3 differs from the perspiration amount measuring devices of the above-mentioned preceding embodiments with respect to the point (a different point) that an inner space 57a where a second thermo-hygro sensor is disposed and an inside air collecting structure 80a are constituted by forming a slope-shaped groove 85 in a flow straightening plate 66. Hereinafter, the embodiment 3 is described by focusing on the different point.

As illustrated in Fig. 9, according to the embodiment 3, the groove 85 is formed in a slope shape on an inner side surface (a flow straightening portion 66a) of the flow straightening plate 66 such that the groove 85 is formed by shaving. The groove 85 is formed such that an entrance of the groove 85 on an upstream side of the flow straightening portion 66a has a large width and a small depth, and an exit of the groove 85 on a downstream side has a small width and a large depth.

A slope 85c of the groove 85 has a depth (so-called degree of shaving) that is increased toward the discharge port 62. In the vicinity of a distal end of the groove 85 on a downstream side, a sensor mounting surface 58 substantially perpendicular to a thickness direction of the fan case 60 is formed, and a lateral wall 59c that is raised from sensor mounting surface 58 is formed (see Fig. 9 (b)). To left and right sides of the lateral wall 59c, lateral walls 59a, 59b that are respectively side walls of the groove 85 are connected. "the inner space 57a in which the second thermo-hygro sensor is disposed" is constituted by these sensor mounting surface 58, lateral walls 59a, 59b, and 59c. The second thermo-hygro sensor 40 is disposed in "the inner space 57a in which the second thermo-hygro sensor is disposed" that is surrounded by three lateral walls 59a, 59b, 59c, and is positioned at the position on an upstream side of inside air as viewed from the edge portion 62a of the discharge port 62. With such a configuration, in the same manner as the embodiment 1, the second thermo-hygro sensor 40 is minimally influenced by disturbance of outside air OA that flows into from the discharge port 62 and hence, the head perspiration amount measurement can be performed more accurately.

Further, with respect to the walls 85a, 85b of the groove, the width of the opening is narrowed toward the edge portion 62a of the discharge port, and is converged toward an edge portion 62a side of the discharge port. The walls 85a, 85b of the groove constitute guide walls 82' that function as the air guides described in the embodiment 2. As a whole, the walls 85a, 85b of the groove form the inside air collecting structure 80a. The second thermo-hygro sensor 40 is disposed in the vicinity of the place COP where the inside airs are collected, and the inside air IA is detected by sensing based on the inside air that represents the whole head H and hence, the head perspiration amount measurement can be performed more accurately.

In this embodiment, the configuration of the perspiration amount measuring device of this embodiment is basically substantially equal to the configuration of the perspiration amount measuring device of the preceding embodiments except for the above-mentioned different point. Accordingly, this embodiment can also acquire the advantageous effects based on the configurations shared in common by these embodiments in the same manner.

[Embodiment 4]

A perspiration amount measuring device (not indicated by symbol) according to an embodiment 4 has basically substantially the same configuration as the perspiration amount measuring devices according to the preceding embodiments. However, the perspiration amount measuring device according to the embodiment 4 differs from the preceding perspiration amount measuring devices with respect to a point (a different point) that the perspiration amount measuring device further includes pressure sensors. Hereafter, the embodiment 4 is described by focusing on the difference.

1. Air volume F relating to discharging of inside air IA by fan 30

In the preceding embodiments, with respect to the calculation of the typical head perspiration amount Y, the description has been made that the head perspiration amount Y is calculated based on the flow-out moisture amount $X_2$, the flow-in moisture amount $X_1$, and the air volume F relating to discharging of the inside air IA by the fan 30. In such calculation, as the air volume F, a fixed value that is obtained by performing reverse calculation based on the specification of abilities of the fan 30 and the fan motor 38, operating conditions of the fan 30 (a rotational speed and the like at the time of operation), an air flow path 10 in which the fan 30 is disposed, the specification of an inner space 60IN of the fan case and the like. In a case where the fan is an axial type fan, the manner of discharging the inside air IA is a one point concentration type and hence, the head perspiration amount Y is not largely affected practically even if the air volume F is treated as a fixed value.

However, with respect to the fan 30 that discharges the inside air IA in the direction that intersects with the rotation axis AX such as, for example, the cross-flow type fan, in general, the opening distance of the discharge port 62 is wide and, at the same time, air supply efficiency is high and hence, it is not so much necessary to rotate the blade 33 at a high speed whereby an air volume per unit area is small and an air speed is also small. Accordingly, the cross-flow type fan is in a state that the outside air OA is liable to easily reversely flow (liable to easily enter the inside of the perspiration amount measuring device). As a result, the head perspiration amount Y is liable to be easily affected by the disturbance of the outside air OA blown into the perspiration amount measuring device from the discharge port 62.

In addition, on a site of a construction work or the like where the perspiration amount measuring device is mainly used, a wearer WR of the perspiration amount measuring device frequently performs a posture change such as temporally leaning forward and again being stood upright and the movement between working places. Further, there is also a case where the wearer WR performs an operation on an upper stair of a building. In such a posture change, movement, and an operation under a particular environment, temporally and transitional air flow is generated around the head H of the wearer WR. When such temporally and transitional air flow enters the discharge port 62 of the fan case 60, the discharge of the inside air IA is temporally interrupted or the discharge of the inside air IA is temporally accelerated. In this manner, strictly speaking, it is considered that an actual air volume F relating to discharging of the inside air IA changes by being affected by the disturbance of the outside air OA from the discharge port 62.

2. Perspiration amount measuring device (not indicated by symbol) according to embodiment 4

In the perspiration amount measuring device according to the embodiment 4, an air volume Fp that reflects the influence of disturbance of the outside air OA described in the above-mentioned 1 is obtained by measuring an atmospheric pressure of the outside air OA and an atmospheric pressure of the inside air IA.

The perspiration amount measuring device according to the embodiment 4 further includes, in addition to the above-mentioned perspiration amount measuring device (the perspiration amount measuring device 1, 2 or the like), a first pressure sensor 75 and a second pressure sensor 76 as indicated by OP1 (Option 1) in Fig. 6. The first pressure sensor 75 is disposed in a place opened to an external field E, and measures an atmospheric pressure of the outside air OA. The second pressure sensor 76 is disposed in the vicinity of the second thermo-hygro sensor 40, and measures an atmospheric pressure of the inside air IA in the vicinity of the second thermo-hygro sensor 40.

It is preferable that the first pressure sensor 75 be disposed in the vicinity of the first thermo-hygro sensor 20. It is preferable that the first pressure sensor 75 and the first thermo-hygro sensor 20 be constituted as one device by being packaged. Further, also with respect to the second pressure sensor 76, it is preferable that the second pressure sensor 76 and the second thermo-hygro sensor 40 be constituted as one device by being packaged.

The perspiration amount measuring device according to the embodiment 4 is configured such that a flow speed v of the inside air IA discharged from the air flow path 10 is obtained based on the deference between a pressure $P_1$ obtained by the first pressure sensor 75 and a pressure $P_2$ obtained by the second pressure sensor 76, an air volume Fp relating to discharging of the inside air IA is obtained based on the flow speed v and a cross-sectional area of the discharge port 62 of the inside air IA, and a perspiration amount Y from the head H is calculated based on the air volume Fp.

The perspiration amount measuring device according to the embodiment 4 can obtain an air volume Fp corresponding to disturbance even when the disturbance of the outside air OA exists in the vicinity of the discharge port 62 due to the above-mentioned configuration and hence, a head perspiration amount Y can be accurately calculated.

3. Calculation of air volume Fp

The calculation of the air volume Fp using the first pressure sensor 75 and the second pressure sensor 76 can be performed in accordance with the following steps, for example, based on the Bernoulli's principle.

The pressure $P_1$ in the vicinity of the first thermo-hygro sensor 20 and the pressure $P_2$ in the vicinity of the second thermo-hygro sensor 40 are measured by the first pressure sensor 75 and the second pressure sensor 76 respectively.

A differential pressure between these $P_1$ and $P_2$ is obtained, and based on the differential pressure, a flow speed v of the inside air IA is obtained from the following formula (4).

$$v=\sqrt{(2(P_1-P_2)/\rho}...(4)$$

In the formula (4), $\rho$ expresses fluid density. In this case, $\rho$ can be determined by the formula (5) assuming $\rho$ as air density. In the formula (5) t expresses a temperature, R expresses a gas constant, and $P_{atm}$ expresses an atmospheric pressure.

$$\rho = P_{atm}/R(t+273.15)\ldots(5)$$

When the flow speed v is obtained by the above-mentioned formula (4) and formula (5), the air volume Fp can be calculated by multiplying the flow speed v by a cross-sectional area of the discharge port 62 of the inside air IA.

In this embodiment, the configurations of the perspiration amount measuring device of this embodiment has basically substantially equal to the configuration of the perspiration amount measuring device of the preceding embodiments except for the above-mentioned different point. Accordingly, this embodiment can also acquire the advantageous effects based on the configurations shared in common by these embodiments in the same manner.

[Embodiment 5]

Fig. 10 is a view describing perspiration amount measuring devices 5, 5' according to the embodiment 5. Fig. 10(a) is a front view of the perspiration amount measuring device 5, and Fig. 10(b) is a right side view of the perspiration amount measuring device 5. Fig. 10(c) is a front view of the perspiration amount measuring device 5', and Fig. 10(d) is a right side view of the perspiration amount measuring device 5'.

The perspiration amount measuring devices 5, 5' according to the embodiment 5 have basically substantially the same configuration as the respective perspiration amount measuring devices according to the embodiments 1 to 3. However, the perspiration amount measuring devices 5, 5' according to the embodiment 5 differ from the respective perspiration amount measuring devices according to the embodiments 1 to 3 with respect to a point (a different point) that the perspiration amount measuring devices 5, 5' further include a flow sensor. Hereinafter, the embodiment 5 is described by focusing on the different point.

1. perspiration amount measuring device 5

The perspiration amount measuring device 5 further includes a first flow sensor 77 in addition to the above-mentioned perspiration amount measuring device (perspiration amount measuring device 1, 2 or the like), as indicated by OP2 (Option 2) in Fig. 6.

The first flow sensor 77 is disposed at a place opened to an external field E, and measures a flow speed of the outside air OA at such an arrangement place. The first flow sensor 77 may be disposed in an outer region of a flow straightening plate 66 in the vicinity of a discharge port 62 of a fan case 60 as indicated by a broken line G1 in Fig 10 (a), for example. Further, the first flow sensor 77 may be disposed in an outer side region of a side plate 68 of the fan case 60 in the vicinity of the discharge port 62 as indicated by a broken line G2.

In this case, the perspiration amount measuring device 5 obtains an air volume Ff relating to discharging of the inside air IA by correcting a predetermined air volume F calculated from the specification of a fan 30 based on a flow speed $v_1$ obtained by the first flow sensor 77, and a perspiration amount from a head H is calculated based on the air volume Ff.

2. perspiration amount measuring device 5'

The perspiration amount measuring device 5' that is another mode of the embodiment 5 further includes a first flow sensor 77 and a second flow sensor 78 in addition to the above-mentioned perspiration amount measuring device (perspiration amount measuring device 1, 2 or the like), as indicated by OP3 (Option 3) in Fig. 6.

The first flow sensor 77 is disposed at a place opened to an external field E, and measures a flow speed of the outside air OA at such an arrangement place. The first flow sensor 77 may be disposed in an outer region of a flow straightening plate 66 in the vicinity of a discharge port 62 of a fan case 60 as illustrated in Fig 10 (b), for example. Further, the first flow sensor 77 may be disposed at the position that corresponds to a broken line G2 in Fig. 10(a).

The second flow sensor 78 is disposed in the vicinity of the second thermo-hygro sensor 40, and measures a flow speed of the inside air IA in the vicinity of the second thermo-hygro sensor 40.

In this case, the perspiration amount measuring device 5' may be configured to obtain a flow speed v of the inside air IA discharged from the air flow path based on a difference between a flow speed $v_1$ obtained by the first flow sensor 77 and a flow speed $v_2$ obtained by the second flow sensor 78, to obtain an air volume Ff relating to discharging of the inside air IA based on the flow speed v and a cross-sectional area of the discharge port of the inside air IA, and to calculate a perspiration amount from the head H based on the air volume Ff.

3. Advantageous effects of perspiration amount measuring devices 5, 5'

The perspiration amount measuring devices 5, 5' according to the embodiment 5 have the above-mentioned configurations. Accordingly, even when disturbance of the outside air OA exists in the vicinity of the discharge port 62, the air volume Ff can be obtained corresponding to the disturbance and hence, a head perspiration amount Y can be accurately calculated.

As the first flow sensor 77 and the second flow sensor 78, for example, a device referred to as an MEMS flow sensor, an MEMS flow rate sensor in a market can be adopted. Such sensors can grasp even a trivial change of air volume as disturbance of the outside air OA and hence, the sensors are suitable as the first flow sensor 77 and the second flow sensor 78.

[Embodiment 6]

The respective perspiration amount measuring devices according to the respective embodiments described above and modifications described later can be used as devices that support the prevention of heat stroke by predicting, rapidly and with high accuracy, a heat stroke onset at a work site based on physiological information (a plurality of biometric data such as a whole body perspiration amount, a whole body salt depletion amount, a heartbeat, a deep body temperature and the like, that are respectively different heat stroke onset mechanisms) including an acquired head perspiration amount. That is, by using the prediction of a heat stroke onset based on the different heat stroke onset mechanism, it is possible to perform the prediction that is multilayered compared to the prediction based on a single mechanism and hence, a risk of occurrence of a heat stroke can be further reduced.

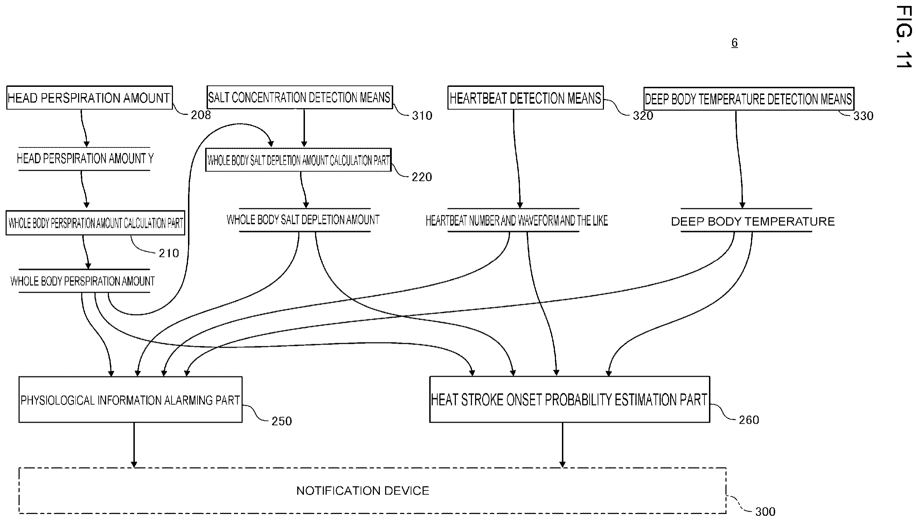

Fig. 11 is an essential part function block diagram for describing essential parts of a perspiration amount measuring device 6 according to an embodiment 6. Some or all functional blocks (for example, a whole body perspiration amount calculation part 210, a whole body salt depletion amount calculation part 220, a physiological information alarming part 250, a heat stroke onset probability estimation part 260 and the like) illustrated in Fig. 11 can be also realized by a processor 71 of a controller 70 or a program executed on the processor 71.

[Alarming based on perspiration amount]

(1) The perspiration amount measuring device 6 according to a first mode includes: a head perspiration amount calculation part 208 that calculates a head perspiration amount Y; a whole body perspiration amount calculation part 210 that calculates a whole body perspiration amount based on the head perspiration amount Y; and a physiological information alarming part 250 (see Fig. 11).

The head perspiration amount calculation part 208 is a functional portion that calculates a perspiration amount from a head (head perspiration amount Y), and the head perspiration amount calculation parts having the configurations described in the embodiments 1 to 5 can be used, for example. The whole body perspiration amount calculation part 210 is a functional portion that calculates a whole body perspiration amount based on the measured perspiration amount from the head (head perspiration amount Y), and the configuration of the whole body perspiration amount calculation part described in [3. Example] of the embodiment 1 can be used.

The physiological information alarming part 250 is a part that performs alarming relating to physiological information (a whole body salt depletion amount, a heartbeat, a deep body temperature described later and the like including a whole body perspiration amount). In the first mode, the physiological information alarming part 250 calculates degree of emergency of taking a measure to cope with a heat stroke and/or a hydration target amount based on the whole body perspiration amount, and outputs the calculated "degree of emergency of a measure to cope with a heat stroke" and/or "hydration target amount" to a notification device 300.

More specifically, the physiological information alarming part 250 detects that numerical values relating to physiological information approaches an abnormal level or has already reached the abnormal level, and calculates "degree of danger of a measure to cope with heat stroke". Further, with respect to perspiration and salt depletion, the physiological information alarming part 250 calculates "a hydration target amount" and "a salt supplement target amount" that are strongly recommendable for recovery from perspiration and salt depletion. Then, the physiological information alarming part 250 performs alarming to a human (in this embodiment, a wearer of the perspiration amount measuring device 6, an administrator who administrates the wearer or the like) via the notification device 300 by presenting such a person with a message "degree of danger of a measure to cope with heat stroke", "a hydration target amount" and the like.

The notification device 300 is an interface device that notifies a human of predetermined information. The notification device 300 may be constituted of, for example, a voice generator, a display device, an information terminal device 306 such as a so-called smartphone or the like (see Fig. 6). The voice generator, the display device or the like may be disposed in a body of the perspiration amount measuring device 6, or may be attached to a helmet 100, for example, as an external part.

Alarming relating to "degree of emergency that requires a person to take a measure to cope with a heat stroke" may be performed by presenting a person with a message informing the degree of danger in stages such as "perform next hydration or salt supplement within 10 minutes", "take a rest within 5 minutes while performing next hydration or salt supplement", "immediately stop an operation and take a rest while performing next hydration or salt supplement" and the like. Alternatively, such alarming may be performed by presenting a person with the degree of danger that is expressed as numerical values under a predetermined rule.

Alarming relating to "hydration target amount" may be performed by presenting a person with a message indicating a specific water amount corresponding to the whole body perspiration amount such as "drink oo ml of water" or "drink half of water filled in a pet bottle of 500 ml".

(2) Further, the perspiration amount measuring device 6 according to the first mode includes a heat stroke onset probability estimation part 260 that estimates the probability of a heat stroke onset, in addition to the whole body perspiration amount calculation part 210.

The heat stroke onset probability estimation part 260 estimates the probability of a heat stroke onset based on the whole body perspiration amount, and outputs the estimated probability of a heat stroke onset to the notification device 300.

Alarming relating to "probability of a heat stroke onset" may be performed by presenting a person with a message informing the onset probability in stages such as "the danger of a heat stroke is increasing (take a rest within 5 minutes)", "you are about to get heat stroke (immediately stop a work and take a rest", "you are in an extremely dangerous state (immediately take a rest while asking help of people around you" and the like. Alternatively, such alarming may be performed by presenting a person with the onset possibility that is expressed as numerical values under a predetermined rule.

(3) The physiological information alarming part 250 and the heat stroke onset probability estimation part 260 can also output various alarming information relating to "degree of emergency of taking a measure to cope with a heat stroke", "hydration target amount", "probability of a heat stroke onset" and the like, together with data of the date on which the alarming information is dispatched and dispatch destinations, to the memory part 72 as an electronic data, and the various alarming information can be recorded and stored in the memory part 72 as an electronic file. Further, the electronic data can be outputted to a memory part of the information terminal device 306, and can be recorded and stored as a file by the information terminal device 306 (particularly, a PC used for an administrator and the like) (see also Fig. 6).

(4) By forming the first mode of the perspiration amount measuring device 6 in such a configuration, a worker (a wearer of the device) can acquire information relating to the specific degrees of "degree of emergency of taking a measure to cope with a heat stroke", "probability of a heat stroke onset" and hence, the worker receives his/her body condition with reality. Further, the worker can acquire information relating to "hydration target amount" by numerical values, the worker can easily take the specific behavior and hence, the worker can increase safety of his/her body and mind.

Further, as viewed from an administrator's side, the administrator can dispatch the instructions on an administrative work for ensuring the safety of a worker (particularly, the instruction relating to the prevention of heat stroke and the instruction of taking a measure to cope with a heat stroke) to the worker in the form of specific contents and hence, the safety of the workers can be enhanced. Further, such dispatching relating to the safety securing operation can be recorded as electronic data and can be used in the safety management.

2. Alarming based on salt depletion amount

(1) The perspiration amount measuring device 6 according to the second mode includes a salt concentration detection means 310, a whole body salt depletion calculation part 220, and a physiological information alarming part 250 (see Fig. 6, Fig. 11).

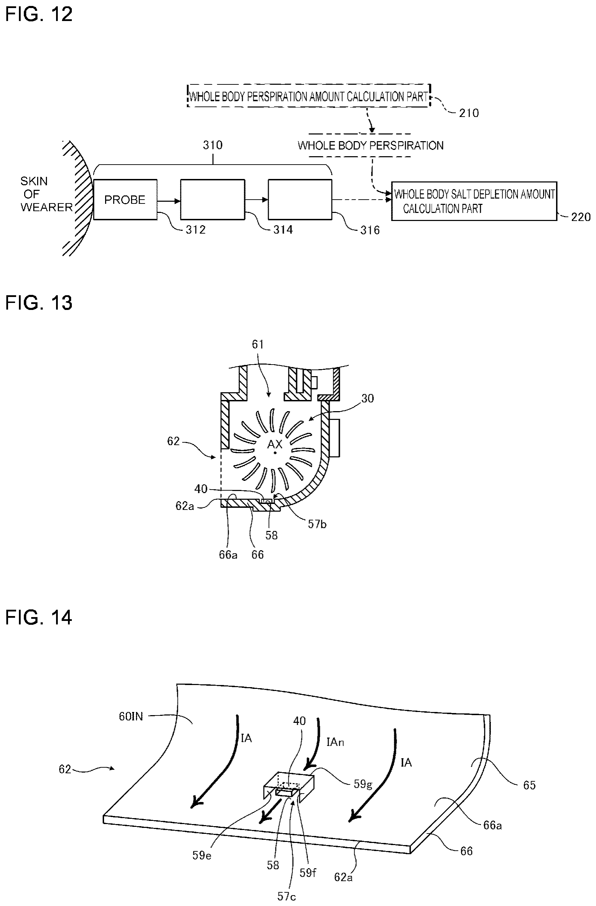

Fig. 12 is a view illustrating a configurational example of the salt concentration detection means 310 according to the embodiment 6. The salt concentration detection means 310 detects salt concentration of a wearer. As illustrated in Fig. 12, the salt concentration detection means 310 includes: a perspiration detection probe 312 that includes a sensor that is brought into contact with a skin of a wearer and measures electric conductivity of perspiration of the wearer; an electric conductivity measurement part 314 that receives the supply of an electric signal detected by the perspiration detection probe 312 and measures electric conductivity based on the electric signal; and a salt concentration calculation part 316 that calculates salt concentration based on electric conductivity that the electric conductivity measurement part 314 outputs. Information relating to salt concentration is supplied to the whole body salt depletion calculation part 220 from the salt concentration detection means 310, and information relating to the whole body perspiration amount is supplied from the whole body perspiration calculation part 210. As the salt concentration detection means 310, for example, a salt concentration measurement device described in JP-A-2021-32738 can be used. The salt concentration detection means 310 is not limited to such a device, and a salt concentration measurement device having the different configuration can be also used.

The whole body salt depletion amount calculation part 220 performs an arithmetic operation based on perspiration salt concentration information that the salt concentration detection means 310 outputs and the whole body perspiration amount information from the whole body perspiration amount calculation part 210, and calculates a whole body salt depletion amount. More specifically, a real-time whole body salt depletion amount is obtained by integrating real-time perspiration salt concentration data and real-time whole body perspiration amount data.

The physiological information alarming part 250 (the physiological information alarming part 250 described in the first mode) calculates a degree of emergency of taking a measure to cope with a heat stroke and/or a salt supplement target amount based on the whole body salt depletion amount calculated by the whole body salt depletion amount calculation part 220, and outputs the calculated "degree of emergency of taking a measure to cope with a heat stroke" and/or "salt supplement target amount" to the notification device 300.

Alarming relating to "degree of emergency of taking a measure to cope with a heat stroke" is performed substantially in the same manner as the embodiment 1 described above.

Alarming relating to "salt supplement target amount" is performed by presenting a specific required salt supplement amount corresponding to the whole body salt depletion amount such as "take salt of oo g" or "take 300 ml of sport drink or more".

(2) Further, the perspiration amount measuring device 6 of the second mode further includes the salt concentration detection unit 310, a whole body salt depletion calculation part, and the heat stroke onset probability estimation part 260 described in the first mode, in addition to the whole body perspiration amount calculation part 210 described in the first mode.

In the second mode, the heat stroke onset probability estimation part 260 is configured to estimate the probability of heat stroke onset based on the whole body perspiration amount and the whole body salt depletion amount, and to output the estimated "probability of heat stroke onset" to the notification device. The main difference between the first mode and the second mode is a point that the heat stroke onset probability estimation part 260 estimates the probability of heat stroke onset by taking into account not only the whole body perspiration amount but also the whole body salt depletion amount.

Alarming relating to "probability of heat stroke onset" is performed substantially in the same manner as the embodiment 1 described above.

The perspiration amount measuring device 6 of the second mode can acquire substantially same advantageous effects as the perspiration amount measuring device 6 of the first mode with respect to the configurations shared in common by both perspiration amount measuring devices 6.

3. Alarming based on heartbeat

(1) The perspiration amount measuring device 6 in a third mode includes a heartbeat detection means 320, and a physiological information alarming part 250 (see Fig. 6, Fig. 11).

The heartbeat detection means 320 detects a heartbeat of a wearer. As the heartbeat detection means 320, a commercially available heartbeat detection device can be suitably used, or a heartbeat detection means dedicated to the present perspiration amount measuring device 6 can be used.

The physiological information alarming part 250 calculates a degree of emergency of taking a measure to cope with a heat stroke and/or heartbeat data based on information that the heartbeat detection means 320 outputs, and outputs the calculated "degree of emergency of taking a measure to cope with a heat stroke" and/or "heartbeat data" to the notification device 300.

Alarming relating to "degree of emergency of taking a measure to cope with a heat stroke" is adopted substantially in the same manner as the embodiment 1 described above.

Alarming relating to "heartbeat data" is performed by presenting a specific content corresponding to information that the heartbeat detection means 320 outputs such as "the number of heartbeats exceeds 120/min", "irregular heartbeat is observed" or the like, for example.

(2) Further, the perspiration amount measuring device 6 of the third mode further includes the heartbeat detection means 320, and the heat stroke onset probability estimation part 260 described in the first mode, in addition to the whole body perspiration amount calculation part 210 described in the first mode.

In the third mode, the heat stroke onset probability estimation part 260 is configured to estimate the probability of heat stroke onset based on the whole body perspiration amount and the information that the heartbeat detection means 320 outputs, and to output the estimated "probability of heat stroke onset" to the notification device. The main difference between the first mode and the third mode is a point that the heat stroke onset probability estimation part 260 estimates the probability of heat stroke onset by taking into account not only the whole body perspiration amount but also the information that the heartbeat detection means 320 outputs.

Alarming relating to "probability of heat stroke onset" is performed substantially in the same manner as the embodiment 1 described above.

The perspiration amount measuring device 6 of the third mode can acquire substantially same advantageous effects as the perspiration amount measuring device 6 of the first mode with respect to the configurations shared in common by both perspiration amount measuring devices 6.

4. Alarming based on deep body temperature

(1) The perspiration amount measuring device 6 in a fourth mode includes a deep body temperature detection means 330, and a physiological information alarming part 250 (see Fig. 6, Fig. 11).

The deep body detection means 330 detects a body temperature of a deep part of a wearer. As the deep body temperature detection means 330, for example, a technique described in Japanese Patent Application No. 2023-222637 that is another patent application filed with respect to the invention that the inventors of the present invention made can be suitably used.

The physiological information alarming part 250 calculates a degree of emergency of taking a measure to cope with a heat stroke and/or deep body temperature data based on information that the deep body temperature detection means 330 outputs, and outputs the calculated "degree of emergency of taking a measure to cope with a heat stroke" and/or "deep body temperature data" to the notification device 300.

Alarming relating to "degree of emergency of taking a measure to cope with a heat stroke" is performed substantially in the same manner as the embodiment 1 described above.

Alarming relating to "deep body temperature data" is performed by presenting a specific content corresponding to information that the deep body temperature detection means 330 outputs such as "the deep body temperature exceeds 40°C" or the like, for example.

(2) Further, the perspiration amount measuring device 6 of the fourth mode further includes the deep body temperature detection means 330, and the heat stroke onset probability estimation part 260 described in the first mode, in addition to the whole body perspiration amount calculation part 210 described in the first mode.

In the fourth mode, the heat stroke onset probability estimation part 260 is configured to estimate the probability of heat stroke onset based on the whole body perspiration amount and the information that the deep body temperature detection means 330 outputs, and to output the estimated "probability of heat stroke onset" to the notification device 300. The main difference between the first mode and the fourth mode is a point that the heat stroke onset probability estimation part 260 estimates the probability of heat stroke onset by taking into account not only the whole body perspiration amount but also the information that the deep body temperature detection means 330 outputs.

Alarming relating to "probability of heat stroke onset" is performed substantially in the same manner as the embodiment 1 described above.

The perspiration amount measuring device 6 of the fourth mode can acquire substantially same advantageous effects as the perspiration amount measuring device 6 of the first mode with respect to the configurations shared in common by both perspiration amount measuring devices 6.

5. Combination of modes

Provided that no contradiction exists technically, the respective modes of the embodiment 6 can be carried out in combination. For example, it is also possible to predict suffering of a heat stroke based on other heat stroke onset mechanism based on one physiological information (also referred to as biological information), for example. The heat stroke onset probability estimation part 260 can also predict suffering of a heat stroke by combining physiological information obtained in the respective embodiments. By performing the prediction in such manners, it is possible to provide heat stroke onset prediction information to a wearer of the perspiration amount measuring device of the present invention rapidly and correctly with high accuracy. It is possible to constitute the helmet 100 and the perspiration amount measuring system together by mounting the perspiration amount measuring device 6 of each mode to the edge portion 102 of the helmet 100.

Although the present invention has been described with reference to the above-mentioned embodiments heretofore, the present invention is not limited to the above-mentioned embodiments. The perspiration amount measuring device according to the present invention can be carried out in various modes without departing from the gist of the present invention. For example, the following modifications are conceivable.

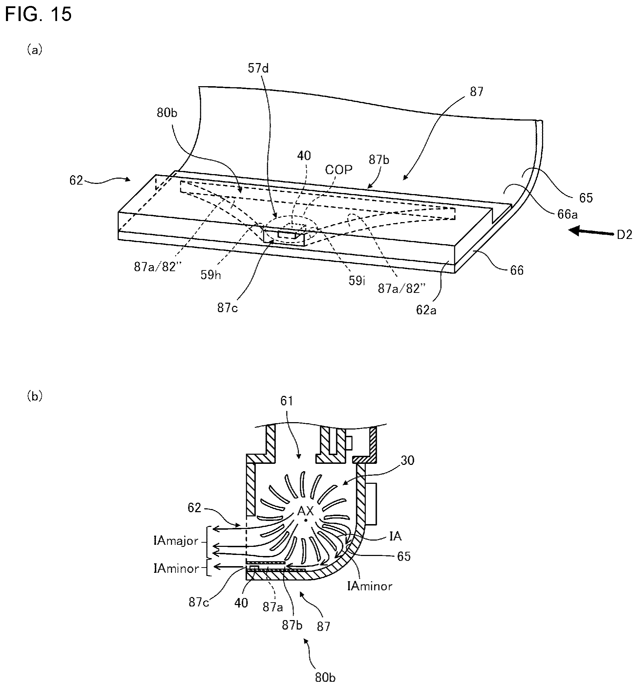

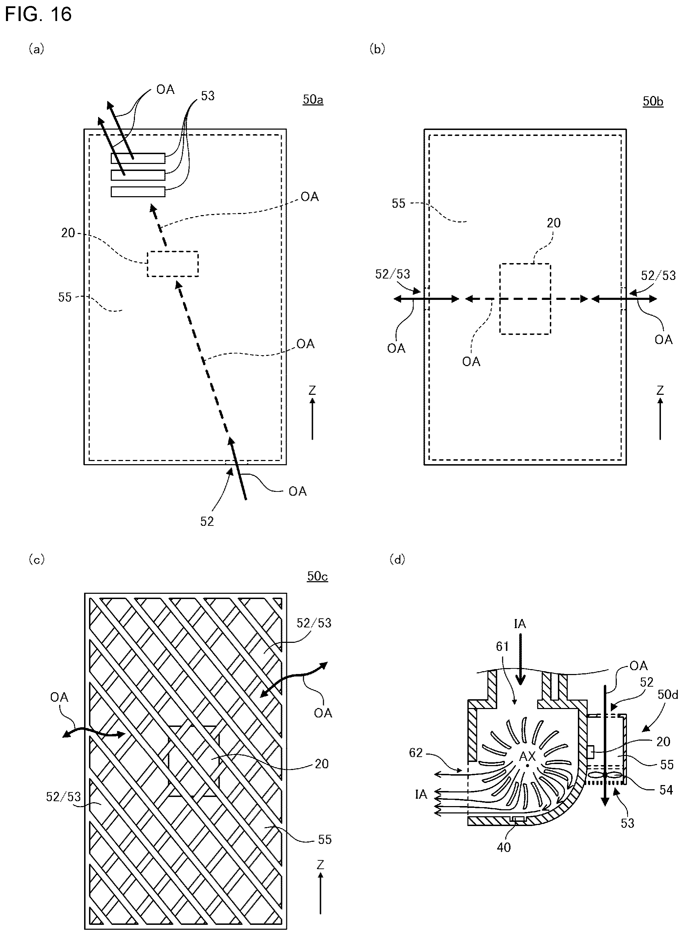

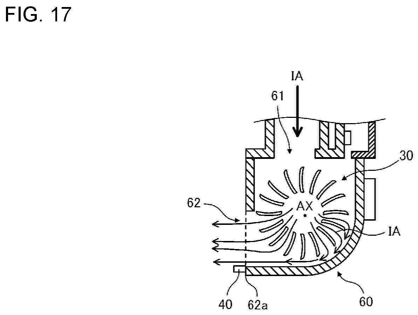

Fig. 13 is a cross-sectional view illustrating an actual structure of an inner space 57b where a second thermo-hygro sensor according to the modification 1 is disposed. Fig. 14 is a perspective view illustrating an actual structure of an inner space 57c where a second thermo-hygro sensor according to a modification 2 is disposed. Fig. 14 is a perspective view of an essential part that corresponds to Fig. 7. Fig. 15 is a view illustrating an actual structure of an inner space 57d where a second thermo-hygro sensor according to a modification 3 is disposed and an inside air collecting structure 80b. Fig. 15(a) is an essential part perspective view corresponding to Fig. 7. Fig. 15(b) is an essential part cross-sectional view corresponding to Fig. 3 as viewed from a direction D2 in Fig. 15(a). Fig. 16 is a view illustrating sensor covers 50a, 50b, 50c, 50d according to a modification 4. Fig. 16(a) to Fig. 16(c) are back views of the sensor covers 50a, 50b, 50c as viewed from a back surface of the perspiration amount measuring device. Fig. 16(d) is an essential part cross-sectional view illustrating the sensor cover 50d. Fig. 17 is an essential part cross-sectional view illustrating a place where a second thermo-hygro sensor according to a modification 5 is disposed.

(1) In the embodiment 1, the recess that is recessed in the thickness direction from the inner side of the flow straightening plate 66 is set as "the inner space 57 in which the second thermo-hygro sensor is disposed", and an outer side of the flow straightening plate 66 that corresponds to the recess is made flat. However, the present invention is not limited to such a configuration. As illustrated in Fig. 13, the outer side of the flow straightening plate 66 that corresponds to the recess may be formed in a protruding shape (modification 1).

(2) In the embodiment 1, the recess that is recessed in the thickness direction from the inner side of the flow straightening plate 66 is set as "the inner space 57 in which the second thermo-hygro sensor is disposed". However, the present invention is not limited to such a configuration. For example, as illustrated in Fig. 14, a tunnel-shaped structure is constituted of lateral walls 59e 59f that are raised from the sensor mounting surface 58 and oppositely face each other; and a spanning surface 59g that is continuously formed with the lateral walls 59e, 59g and oppositely faces the sensor mounting surface 58, and such a tunnel-shaped structure may be set as "the inner space 57 in which the second thermo-hygro sensor is disposed". With respect to the tunnel-shaped structure, portions of the tunnel-shaped structure that correspond to an upstream side and a lower stream side of inside air IAn are opened so that the inside air IAn can smoothly flow without receiving resistance. (modification 2)

(3) In the embodiment 2 and the embodiment 3, the inside air collecting structures 80, 80a are formed by constructing the rib 83 and the slope-shaped groove 85. However, the present invention is not limited to such configurations. For example, a guide casing 87 illustrated in Fig. 15 is introduced, and partition walls 87a of the guide casing 87 may be set as guide walls 82'. With such a configuration, inside air $IA_{minor}$ are taken in from an inlet 87b of the guide casing 87 having a wide distance, are merged and mixed in the inside sandwiched between two partition walls 87a, and are discharged from an outlet 87c. It is preferable that "a place COP where the inside airs are collected" be disposed in the vicinity of the outlet 87c. It is preferable that portions in the vicinity of the lateral walls 59h, 59i be set as terminals of the partition walls 87a, and a portion in the vicinity of the outlet 87 inside the guide casing 87 be set as "the inner space 57d in which the second thermo-hygro sensor is disposed", and the second thermo-hygro sensor 40 be disposed in the space (modification 3).

(4) In the respective embodiments and modifications described heretofore, from the space 55 where the flow of outside air OA in the sensor cover 50 is generated, the outside air OA is discharged through the communicating portion 95. However, the present invention is not limited to such a configuration. For example, as illustrated in Fig. 16(a) to Fig.16(d), the configuration may be adopted where, without using the communicating portion 95, outside air outlet ports 53 are formed in the sensor covers 50a to 50d besides the outside air intake port 52, the outside air OA is taken from the outside air intake port 52, flows in the vicinity of the first thermo-hygro sensor 20, and is discharged from the outside air outlet port 53. In the sensor cover 50d illustrated in Fig. 16 (d), the sensor-use fan 54 is provided for forcibly generating the flow of the outside air OA (modification 4).

(5) In the respective embodiments and modifications described heretofore, the description has been made with respect to the example where the second thermo-hygro sensor 40 is disposed in the fan case 60. However, the present invention is not limited to such a case. For example, as illustrated in Fig. 17, the second thermo-hygro sensor 40 may be disposed outside at a position extremely close to the discharge port 62 of the fan case 60 (modification 5). Also in this case, the second thermo-hygro sensor 40 is treated as being "disposed in the flow of inside air" so that the modification 5 can acquire the manner of operation and advantageous effects of the present invention.

Reference signs list

1, 2, 5, 5': perspiration amount measuring device
9: perspiration amount measuring system
10: air flow path

EP 4 678 052 A1

12: opening
20: first thermo-hygro sensor
30: fan
33: blade
34: annular frame body
35: bladed wheel impeller
36: shaft
37: fan body
38: fan motor
39: bearing
40: second thermo-hygro sensor
50, 50a, 50b, 50c, 50d: sensor cover
52: outside air intake port
53: outside air outlet port
54: sensor-use fan
55: space where the flow of outside air OA is generated
57, 57a, 57b, 57c, 57d: inner space where second thermo-hygro sensor is disposed
58: sensor mounting surface
59, 59a, 59b, 59c, 59d, 59e, 59f, 59g, 59h, 59i: lateral wall
60: fan case
601N: inner space of fan case
61: suction port
61a: edge portion of suction port
62: discharge port
62a: edge portion of discharge port
64: inner wall (of fan case)
65: curved surface portion
66: flow straightening plate
66a: flow straightening portion
67: shut plate
68: side plate
70: control unit
71: processor
72: memory part
72a: memory
72b: storage
73: communication I/F
74: input/output I/F
75: first pressure sensor
76: second pressure sensor
77: first flow sensor
78: second flow sensor
80, 80a, 80b: inside air collecting structure
82: 82',82": guide wall,
83, 83a, 83b,83c: rib
85: (slope-shaped) groove
85a, 85b: wall of groove
85c: slope
87: guide casing
87a: partition wall (of guide casing)
87b: inlet (of guide casing)
87c: outlet (of guide casing)
92: clip
92a: clip inner peripheral member
92b: clip outer peripheral member
92: clip side peripheral member
94: shield member
95: communicating portion

23

100: helmet
101: outer shell (of helmet)
102: edge portion (of helmet)
103: intermediate covering member
110: helmet inside air flow path
204: inflow moisture amount calculation part
206: outflow moisture amount calculation part
208: head perspiration amount calculation part
210: whole body perspiration amount calculation part 220: whole body salt depletion amount calculation part
250: physiological information alarming part,
260: heat stroke onset probability estimation part
300: notification device
306: information terminal device
310: salt concentration detection means
312: perspiration detection probe
314: electric conductivity measurement part
316: salt concentration calculation part
320: heartbeat detection means
330: deep body temperature detection means

**Claims**

1. A perspiration amount measuring device that is mountable on an edge portion of a helmet and is capable of measuring a perspiration amount from a head of a wearer of the helmet, the perspiration amount measuring device comprising:

   an air flow path through which inside air that is air containing vapor generated from the head flows;
   a first thermo-hygro sensor that is disposed at a place opened to an external field, and measures a temperature and a relative humidity of outside air that is air in the external field that is taken in from a place on a side opposite to the wearer as viewed from the air flow path;
   a fan that includes: a plurality of blades; and a fan case that houses the plurality of blades therein and is provided with a suction port and a discharge port, the plurality of blades being rotatable about a rotation axis so as to suck the inside air in the air flow path from the suction port and to discharge the inside air to an outside from the discharge port; and
   a second thermo-hygro sensor that is disposed in a flow of the inside air generated due to an operation of the fan, and measures a temperature and a relative humidity of the inside air, wherein
   the fan discharges the inside air in a direction that intersects with the rotation axis.

2. The perspiration amount measuring device according to claim 1, wherein
   the second thermo-hygro sensor is disposed at a position on an upstream side of the inside air as viewed from an edge portion of the discharge port.

3. The perspiration amount measuring device according to claim 1 or 2, wherein
   an inner space where the second thermo-hygro sensor is disposed is defined by a sensor mounting surface that is substantially perpendicular to a thickness direction of the fan case and a lateral wall that is raised from the sensor mounting surface.

4. The perspiration amount measuring device according to any one of claims 1 to 3, wherein

   the fan is configured such that, as viewed in a direction perpendicular to the rotation axis, an air flow that includes the inside airs corresponding to respective portions that are generated from a plurality of arbitrary portions of the head of the wearer is sucked into the inside of the fan case from the suction port while allowing the inside airs to maintain a parallel relationship with each other and is discharged from the discharge port while allowing the inside airs to maintain the parallel relationship with each other,
   at a position in the inside of the fan case and close to the discharge port, an inside air collecting structure is provided, and the inside air collecting structure is configured such that, due to an air guide that guides a flow of air, with respect to some inside airs among the inside airs to be discharged, the inside airs that are allowed to flow parallel to each other and correspond to the respective portions are mixed together and are collected as the inside airs move toward the discharge port, and

the second thermo-hygro sensor is disposed in the vicinity of a place in the inside air collecting structure where the inside airs are collected.

5. The perspiration amount measuring device according to claim 4, wherein

the inside air collecting structure includes a plurality of guide walls as the air guide,

the guide walls have surfaces in a direction that intersects with a direction that air flow of the inside airs enters the inside air collecting structure from upstream of the inside air collecting structure, and the surface of the plurality of guide walls are converged toward a place where the inside airs are collected.

6. The perspiration amount measuring device according to any one of claims 1 or 5, further comprising:

a first pressure sensor that is disposed at the place opened to the external field, and measures an atmospheric pressure of the outside air; and

a second pressure sensor that is disposed in a vicinity of the second thermo-hygro sensor, and measures an atmospheric pressure of the inside air in a vicinity of the second thermo-hygro sensor, wherein

a flow speed v of the inside air discharged from the air flow path is obtained based on a difference between a pressure $P_1$ obtained by the first pressure sensor and a pressure $P_2$ obtained by the second pressure sensor, an air volume $F_p$ relating to discharging of the inside air is obtained based on the flow speed v and a cross-sectional area of the discharge port of the inside air, and a perspiration amount from the head is calculated based on the air volume $F_p$.

7. The perspiration amount measuring device according to any one of claims 1 to 5, further comprising a first flow sensor that is disposed at the place opened to the external field and measures a flow speed of the outside air at the place, wherein

the perspiration amount measuring device obtains a flow volume Ff relating to discharging of the inside air by correcting a predetermined air volume F calculated from a specification of the fan based on a flow speed $v_1$ obtained by the first flow sensor, and calculates a perspiration amount from the head based on the air volume Ff.

8. The perspiration amount measuring device according to any one of claims 1 to 5, further comprising:

a first flow sensor that is disposed at the place opened to the external field and measures a flow speed of the outside air at the place; and

a second flow sensor that is disposed in a vicinity of the second thermo-hygro sensor, and measures a flow speed of the inside air in the vicinity of the second thermo-hygro sensor, wherein

a flow speed v of the inside air discharged from the air flow path is obtained based on a difference between a flow speed $v_1$ obtained by the first flow sensor and a flow speed $v_2$ obtained by the second flow sensor, an air volume Ff relating to discharging of the inside air is obtained based on the flow speed v and a cross-sectional area of the discharge port of the inside air, and calculates a perspiration amount from the head based on the air volume Ff.

9. The perspiration amount measuring device according to any one of claims 1 to 5 further comprising:

a whole body perspiration amount calculation part that calculates a whole body perspiration amount based on a perspiration amount from the head that is measured; and

a physiological information alarming part that performs alarming relating to physiological information, wherein the physiological information alarming part calculates a degree of emergency of taking a measure to cope with a heat stroke and/or a hydration target amount based on the whole body perspiration amount, and outputs the degree of emergency of taking a measure to cope with a heat stroke and/or the hydration target amount that are/is calculated to a notification device.

10. The perspiration amount measuring device according to any one of claims 1 to 5 further comprising:

a whole body perspiration amount calculation part that calculates a whole body perspiration amount based on a perspiration amount from the head that is measured; and

a heat stroke onset probability estimation part that estimates probability of a heat stroke onset, wherein the heat stroke onset probability estimation part estimates probability of the heat stroke onset based on the whole body perspiration amount, and outputs the probability of the heat stroke onset that is estimated to a notification device.

11. The perspiration amount measuring device according to any one of claims 1 to 5 further comprising:

a salt concentration detection means that detects salt concentration of the wearer;
a whole body salt depletion amount calculation part that calculates a whole body salt depletion amount based on information that the salt concentration detection means outputs; and
a physiological information alarming part that performs alarming relating to physiological information, wherein the physiological information alarming part calculates a degree of emergency of taking a measure to cope with a heat stroke and/or a salt supplement target amount based on the whole body salt depletion amount, and outputs the degree of emergency of taking a measure to cope with a heat stroke and/or the salt supplement target amount that is calculated to a notification device.

12. The perspiration amount measuring device according to any one of claims 1 to 5 further comprising:

a whole body perspiration amount calculation part that calculates a whole body perspiration amount based on a perspiration amount from the head that is measured;
a salt concentration detection means that detects salt concentration of the wearer;
a whole body salt depletion amount calculation part that calculates a whole body salt depletion amount based on information that the salt concentration detection means outputs; and
a heat stroke onset probability estimation part that estimates probability of a heat stroke onset, wherein the heat stroke onset probability estimation part estimates the probability of the heat stroke onset based on the whole body perspiration amount and the whole body salt depletion amount, and outputs the probability of the heat stroke onset that is estimated to a notification device.

13. The perspiration amount measuring device according to any one of claims 1 to 5 further comprising:

a heartbeat detection means that detects a heartbeat of the wearer; and
a physiological information alarming part that performs alarming relating to physiological information, wherein the physiological information alarming part calculates a degree of emergency of taking a measure to cope with a heat stroke and/or heartbeat data based on information that the heartbeat detection means outputs, and outputs the degree of emergency of taking a measure to cope with a heat stroke and/or the heartbeat data that is calculated to a notification device.

14. The perspiration amount measuring device according to any one of claims 1 to 5 further comprising:

a whole body perspiration amount calculation part that calculates a whole body perspiration amount based on a perspiration amount from the head that is measured;
a heartbeat detection means that detects a heartbeat of the wearer; and
a heat stroke onset probability estimation part that estimates probability of a heat stroke onset, wherein the heat stroke onset probability estimation part estimates probability of a heat stroke onset based on information on the whole body perspiration amount and information that the heartbeat detection means outputs, and outputs the probability of a heat stroke onset that is estimated to a notification device.

15. The perspiration amount measuring device according to any one of claims 1 to 5 further comprising:

a deep body temperature detection means that detects a deep body temperature of the wearer; and
a physiological information alarming part that performs alarming relating to physiological information, wherein the physiological information alarming part calculates a degree of emergency of taking a measure to cope with a heat stroke and/or deep body temperature data based on information that the deep body temperature detection means outputs, and outputs the degree of emergency of taking a measure to cope with a heat stroke and/or the deep body temperature data that are calculated to a notification device.

16. The perspiration amount measuring device according to any one of claims 1 to 5 further comprising:

a whole body perspiration amount calculation part that calculates a whole body perspiration amount based on a perspiration amount from the head that is measured;

a deep body temperature detection means that detects a deep body temperature of the wearer; and
a heat stroke onset probability estimation part that estimates probability of a heat stroke onset, wherein the heat stroke onset probability estimation part estimates the probability of the heat stroke onset based on the whole body perspiration amount and the information that the deep body temperature detection means outputs, and outputs the probability of the heat stroke onset to a notification device.

17. The perspiration amount measuring device according to any one of claims 1 to 16, further comprising a sensor cover that covers the first thermo-hygro sensor.

18. The perspiration amount measuring device according to claim 17, wherein
the sensor cover includes an outside air intake port and an outside air outlet port.

19. The perspiration amount measuring device according to claim 18, wherein
the first thermo-hygro sensor is disposed in a space where a flow of the outside air is generated.

20. The perspiration amount measuring device according to claim 19, further comprising:
a shield member that has a communicating portion between the air flow path and a space where the first thermo-hygro sensor is disposed.

21. The perspiration amount measuring device according to claim 20, wherein
the space where the first thermo-hygro sensor is disposed is formed such that the outside air flows with a substantially constant air volume in a vicinity of the first thermo-hygro sensor.

22. The perspiration amount measuring device according to claim 21, wherein
the first thermo-hygro sensor is disposed on an upstream side of the communicating portion and in a vicinity of the communicating portion when viewed by focusing on a flow of the outside air.

23. The perspiration amount measuring device according to any one of claims 1 to 22, wherein
a position at which the fan is disposed is set such that a destination to which air is discharged by the fan is located in a region from a back of the head to a back of a neck of the wearer in a state where the perspiration amount measuring device is mounted on an edge portion of the helmet.

24. The perspiration amount measuring device according to any one of claims 1 to 23, wherein

defining a space formed between an outer shell of the helmet and the head of the wearer as a helmet inside air flow path,
the perspiration amount measuring device is a device that is configured:

to calculate a moisture amount per unit volume as an inflow moisture amount by measuring a temperature and a relative humidity of the air in the external field that flows into the helmet inside air flow path using the first thermo-hygro sensor,
to calculate a moisture amount per unit volume as an outflow moisture amount by measuring a temperature and a relative humidity of the inside air generated in the helmet inside air flow path using the second thermo-hygro sensor,
to calculate a head perspiration amount that is a perspiration amount from the head based on the inflow moisture amount, the outflow moisture amount and an air volume relating to discharging of the inside air by the fan, and
to estimate a whole body perspiration amount based on the head perspiration amount that is calculated.

25. A perspiration amount measuring system that includes: a helmet, and the perspiration amount measuring device according to any one of claims 1 to 24 that is mounted on an edge portion of the helmet.

FIG. 1

FIG. 2

FIG. 3

FIG. 4

(a)

(b)

FIG. 5

CALCULATED VALUE OF HEAD PERSPIRATION AMOUNT EQUIVALENT AMOUNT
DEPENDING ON PLACES WHERE FIRST THERMO-HYGRO SENSOR IS DISPOSED
(SIMULATION)

FIG. 6

EP 4 678 052 A1

CONTROLLER

70

71 — PROCESSOR

74 — INPUT AND OUTPUT I/F

72 — MEMORY
72a
STORAGE
72b

COMMUNICATION I/F
73

BS

20 — FIRST THERMO-HYGRO SENSOR

40 — SECOND THERMO-HYGRO SENSOR

75 — FIRST PRESSURE SENSOR

OP1
76 — SECOND PRESSURE SENSOR

OP3 OP2 77 — FIRST FLOW SENSOR

78 — SECOND FLOW SENSOR

310 — SALT CONCENTRATION DETECTION MEANS

320 — HEARTBEAT DETECTION

330 — DEEP BODY TEMPERATURE DETECTION MEANS

FAN
30

NOTIFICATION MEANS — 300

BAT

POWER

BATTERY

NW

306/300

306/300

FIG. 7

FIG. 8

(a)

(b)

(c)

FIG. 9

(a)

(b)

FIG. 10

(a)

(b)

(c)

(d)

FIG. 11

EP 4 678 052 A1

6

| HEAD PERSPIRATION AMOUNT | 208 | SALT CONCENTRATION DETECTION MEANS | 310 | HEARTBEAT DETECTION MEANS | 320 | DEEP BODY TEMPERATURE DETECTION MEANS | 330 |

HEAD PERSPIRATION AMOUNT Y

WHOLE BODY SALT DEPLETION AMOUNT CALCULATION PART — 220

WHOLE BODY PERSPIRATION AMOUNT CALCULATION PART — 210

WHOLE BODY SALT DEPLETION AMOUNT

HEARTBEAT NUMBER AND WAVEFORM AND THE LIKE

DEEP BODY TEMPERATURE

WHOLE BODY PERSPIRATION AMOUNT

PHYSIOLOGICAL INFORMATION ALARMING PART — 250

HEAT STROKE ONSET PROBABILITY ESTIMATION PART — 260

NOTIFICATION DEVICE — 300

38

FIG. 12

WHOLE BODY PERSPIRATION AMOUNT CALCULATION PART ··· 210

WHOLE BODY PERSPIRATION

SKIN OF WEARER

310

PROBE — 312 — 314 — 316

WHOLE BODY SALT DEPLETION AMOUNT CALCULATION PART — 220

FIG. 13

61
30
62
AX
40
62a
57b
58
66a 66

FIG. 14

60IN
40
IA
IAn
IA
65
62
59g
66a
66
59e
58 59f 62a
57c

FIG. 15

(a)

(b)

## FIG. 16

(a)

(b)

(c)

(d)

FIG. 17

## INTERNATIONAL SEARCH REPORT

| International application No. |
|---|
| **PCT/JP2024/007563** |

| A. | CLASSIFICATION OF SUBJECT MATTER |
|---|---|

*A42B 3/28*(2006.01)i; *A42B 3/04*(2006.01)i; *F04D 17/04*(2006.01)i
FI:   A42B3/28; F04D17/04 E; A42B3/04

According to International Patent Classification (IPC) or to both national classification and IPC

| B. | FIELDS SEARCHED |
|---|---|

Minimum documentation searched (classification system followed by classification symbols)

A42B3/28; A42B3/04; F04D17/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2020/184686 A1 (PUBLIC UNIVERSITY CORPORATION SUWA UNIV. OF SCIENCE FOUNDATION) 17 September 2020 (2020-09-17) paragraphs [0007]-[0175], fig. 1-4 | 1-25 |
| A | JP 62-199811 A (SANYO ELECTRIC CO., LTD.) 03 September 1987 (1987-09-03) p. 1, right column, line 15 to p. 3, line 10, fig. 1-7 | 1-25 |
| A | JP 2019-183373 A (TOKYO UNIVERSITY OF SCIENCE FOUNDATION) 24 October 2019 (2019-10-24) paragraphs [0029]-[0114], fig. 1-4 | 1-25 |

☐ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **23 April 2024** | **14 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/007563**

| Patent document cited in search report | | | Publication date (day/month/year) | Patent family member(s) | Publication date (day/month/year) |
|---|---|---|---|---|---|
| WO | 2020/184686 | A1 | 17 September 2020 | (Family: none) | |
| JP | 62-199811 | A | 03 September 1987 | (Family: none) | |
| JP | 2019-183373 | A | 24 October 2019 | (Family: none) | |

Form PCT/ISA/210 (patent family annex) (July 2022)

**REFERENCES CITED IN THE DESCRIPTION**

**Patent documents cited in the description**

- WO 2020184686 A
- JP 2022064656 A
- JP 2023003297 W

- JP 2021032738 A
- JP 2023222637 A

**Non-patent literature cited in the description**

- **SHINSUKE SAWASAKI**. Study on Thermal Evaluation Method for Hot Outdoor Environment. *Seisan Kenkyu*, 2006, vol. 58 (3), 323-327

- Development of a Helmet Device Capable of Measuring Perspiration during Activity and Possibility of New Index for the Early Detection of Heat Stroke. **TSUKASA KOSUDA**. Journal of Japan Institute of Electronics Packaging, General Incorporated Association. Japan Institute of Electronics Packaging, 2021, vol. 24, 541-550